(19) Europäisches Patentamt
European Patent Office
Office européen des brevets

(11) Publication number: 0 273 349 A2

# (12) EUROPEAN PATENT APPLICATION

(21) Application number: **87118947.8**

(22) Date of filing: **21.12.87**

(51) Int. Cl.⁴: **C07B 57/00**, C07D 211/90, C07D 453/04

Claim for the following Contracting State: ES.

(30) Priority: **22.12.86 US 945760**

(43) Date of publication of application: **06.07.88 Bulletin 88/27**

(84) Designated Contracting States: **AT BE CH DE ES FR GB GR IT LI LU NL SE**

(71) Applicant: **LABORATOIRES SYNTEX S.A.**
**20, Rue Jean Jaurès**
**F-92807 Puteaux Cedex(FR)**

(72) Inventor: **Genain, Gilles**
**3 rue Edouard Branly**
**F-92130 Issy-les-Moulineaux(FR)**

(74) Representative: **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**D-8000 München 40(DE)**

## (54) Resolution of 1,4-dihydropyridine derivatives.

(57) Optically active compounds of the formula

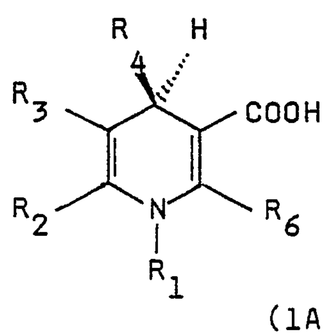

(1A)

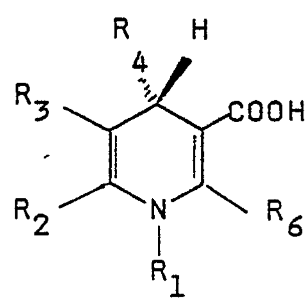

(2A)

or a salt thereof
wherein
$R_1$ is H or lower alkyl;
$R_2$ and $R_6$ are each independently lower alkyl, aryl, or arylalkyl;
$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl;
$R_4$ is aryl, heterocyclyl, or fused-ring heterocyclyl, optionally substituted with one, two, or three halo, $NO_2$, CN, lower alkyl, lower alkoxy, lower alkylamino, $CF_3$, $OCH_2F$, or $OCF_3$;
are formed from a salt mixture of the compound with a resolving agent in a hot solvent mixture of an organic solvent and water.

EP 0 273 349 A2

## RESOLUTION OF 1,4-DIHYDROPYRIDINE DERIVATIVES

This invention relates to a process for resolving 1,4-dihydropyridine derivatives into optically active isomers or a salt thereof, to optically active 1,4-dihydropyridine derivative salts, and to a process for preparing pharmaceuticals from the resolved 1,4-dihydropyridine derivatives.

Certain 4-aryl-1,4-dihydropyridine derivatives are known calcium entry agonists and antagonists. See, for example, U.S. Patent Nos. 3,485,847, 4,044,141, and 4,595,690. It has been found that resolved optical isomers of dihydropyridine derivatives usually have biological activity which differs from the activity of racemic mixtures thereof. It is known that for many biologically active dihydropyridine derivatives the isomer which has an (S) configuration at C4 in the dihydropyridine ring has greater calcium entry blocking activity that the (R) isomer. The (R) isomer of the compound 2,6-dimethyl-3-nitro-4-(2-trifluoromethylphenyl)-5-carbomethoxy-1,4-dihydropyridine displays calcium entry antagonism, while the (S) isomer is a calcium entry facilitator. It is to be appreciated that calcium entry agonists and calcium entry antagonists are each therapeutic in the appropriate circumstances.

Compounds with the (S) configuration may be prepared from (S)-2,6-dialkyl-3-carboalkoxy-4-aryl-5-carboxy-1,4-dihydropyridine derivatives of formula 1. This (S) isomer has been prepared following the method of T. Shibanuma, et al., Chem. Pharm. Bull., 28, 2809-2812 (1980), by resolving 1-ethoxymethyl-2,6-dimethyl-3-carbomethoxy-4-(3-nitrophenyl)-5-carboxy-1,4-dihydropyridine using cinchonidine in methanol. However, Shibanuma's method requires that one protect the dihydropyridine nitrogen using chloromethyl ethyl ether (a flammable lachrymator) in the presence of sodium hydride, monosaponify, and later remove the protecting group by acid hydrolysis. This protection and deprotection reduces the ultimate yield of resolved dihydropyridine derivative.

Other methods of achieving optical resolution are described in EP 26,317, FR 2,528,431, and FR 2,523,128. These methods avoid the need to protect and deprotect the dihydropyridine nitrogen, but require the addition of special ester groups to achieve derivatives that may be separated. For example, EP 26,317 discloses a method for resolving dihydropyridine derivatives by esterifying a dihydropyridine acid with (R)-2-phenyl-2-methoxyethanol, separating the resulting diastereomers by chromatography, and removing the 2-phenyl-2-methoxyethoxy group to provide the resolved dihydropyriding acid. Of course, each esterification and saponification detracts from the overall yield of the resolution, and chromatographic processes are usually to be avoided in pharmaceutical manufacturing.

I have now discovered a more efficient, effective, and less expensive method for preparing optically active mono-esterified dihydropyridine derivatives or a salt thereof directly, which dispenses with the need for protecting groups and ester groups, and which is easily practiced at an industrial scale. The compounds of formulas 1, 2, 1A, or 2A are useful for preparing calcium entry modulators of therapeutic value.

One aspect of the invention is a process for preparing optically active compounds of formula (X):

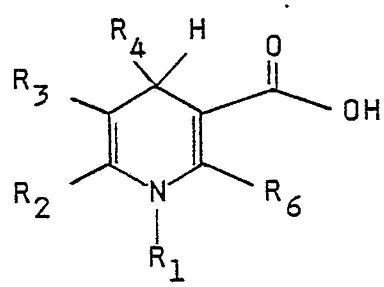

(X)

or a salt thereof
wherein
$R_1$ is H or lower alkyl;
$R_2$ and $R_6$ are each independently lower alkyl, aryl, or arylalkyl;
$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl;
$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$.

Another aspect of the invention is a process for preparing a 1,4-dihydropyridine derivative having a

carboxylic acid or carboxylic acid derivative function in position 5 of the dihydropyridine ring and an aryl, heterocyclyl, or fused ring heterocyclyl, optionally substituted with one, two or three substituents in position 4 of the dihydropyridine ring, or a salt thereof, which process comprises forming a salt mixture of a compound of formula (X)

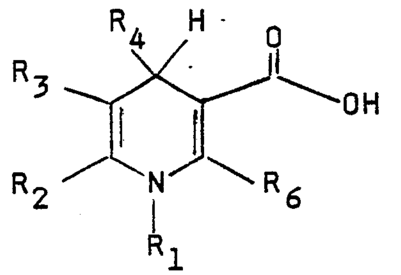

(X)

or a salt thereof

wherein

$R_1$ is H or lower alkyl;

$R_2$ and $R_6$ are each independently lower alkyl, aryl, or arylalkyl;

$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl; and

$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$;

with a resolving agent in a solvent selected from dimethyl formamide, ethanol, and acetonitrile, optionally in admixture with water, at a temperature between 50°C and the reflux temperature of said solvent, optionally adding water during this elevated temperature, such that the ratio of 1,4-dihydropyridine derivative to resolving agent is about 1:2 to about 2:1 and the mixture contains about 10% to about 50% water, wherein said salt mixture comprises

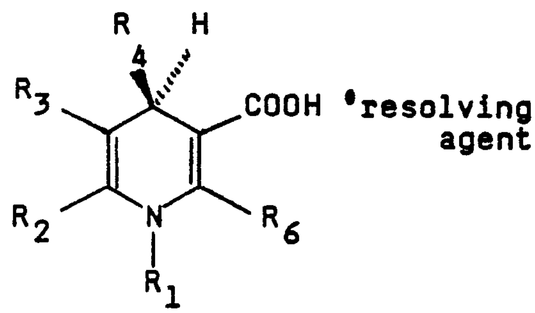

(1)

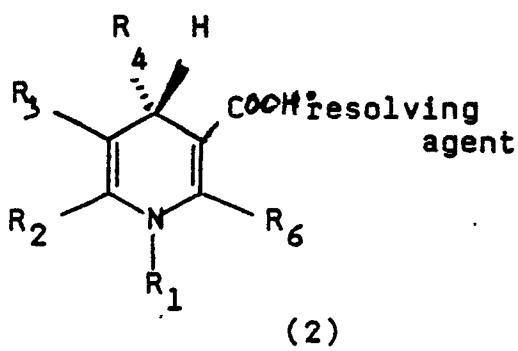

(2)

or a salt thereof

wherein $R_1$, $R_2$, $R_3$, $R_4$, and $R_6$ are as defined above.

Another aspect of the invention is a process for the preparation of 1,4-dihydropyridine derivatives wherein said 1,4-dihydropyridine derivative is a compound of the formula (1A) or (2A)

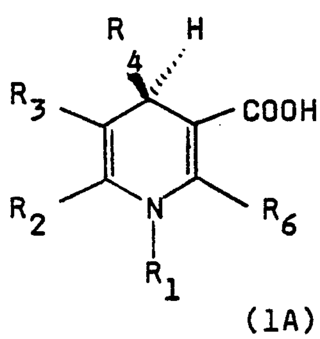

(1A)

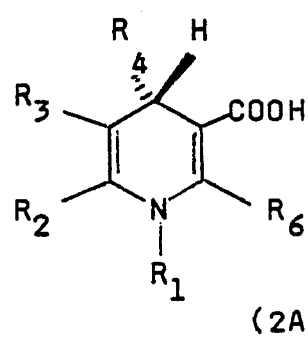

(2A)

or a salt thereof
wherein
$R_1$ is H or lower alkyl;
$R_2$ and $R_6$ are each independently lower alkyl, aryl, or arylalkyl;
$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl;
$R_4$ is aryl, heterocyclyl, or fused-ring heterocyclyl, optionally substituted with one, two, or three halo, $NO_2$, CN, lower alkyl, lower alkoxy, lower alkylamino, $CF_3$, $OCH_2F$, or $OCF_3$.

Another aspect of the invention is a process for the preparation of 1,4-dihydropyridine derivatives, which process further comprises

a) separating the compound represented by the formula (1) from the compound represented by the formula (2), and

b) optionally purifying the isolated compound of formula (1) or (2).

Another aspect of the invention is a process for the preparation of 1,4-dihydropyridine derivatives, which process further comprises cleaving a compound of formula (1) or (2) to form a compound of the formula (1A) or (2A).

Another aspect of the invention is a compound of the formula (1) or (2)

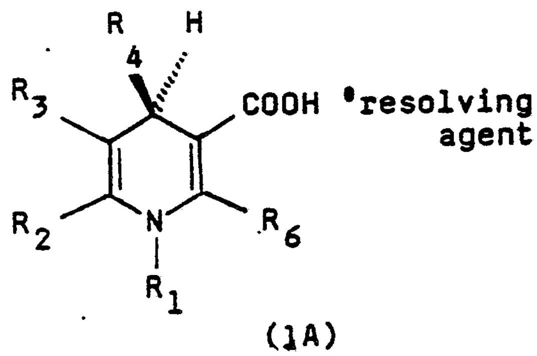
(1A)

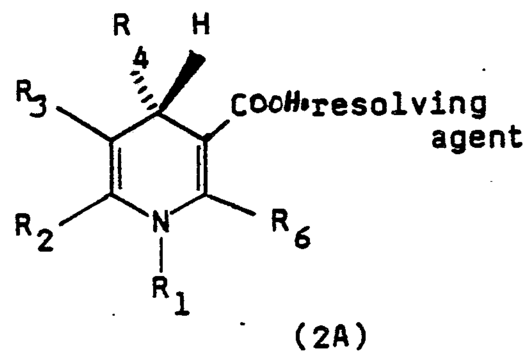
(2A)

or a salt thereof
wherein
$R_1$ is H or lower alkyl;
$R_2$ and $R_6$ are each independently lower alkyl, aryl, or arylalkyl;
$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl or arylalkyl;
$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$; and
resolving agent comprises an optically active amine base such as cinchonidine, cinchonine, quinine, quinidine, strychnine, brucine, morphine, D-α-phenylethylamine, L-arginine, dehydroabietylamine, cinchonicine, L-2-amino-1-propanol, D-amphetamine, glucosamine, conessine, anabasine, D-ephedrine, or an N-alkyl-D-glucamine wherein alkyl is C1 to C10.

Another aspect of the invention is a process for the preparation of 1,4-dihydropyridine derivatives of the formula (3)

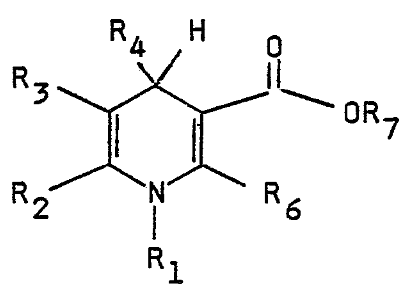
(3)

or a salt thereof
wherein

$R_1$ is H or lower alkyl;

$R_2$ and $R_6$ are each independently lower alkyl, aryl, or arylalkyl;

$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl;

$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$;and

$R_7$ is an alcohol derivative of the general formula $HOR_8$ wherein $R_8$ is $-O(CH_2)_nR_9$ wherein n is 2 or 3 and $R_9$ is NR′R″ wherein R′ is lower alkyl and R″ is arylalkyl, phenyl substituted in the para position with a 2,3-dihydroxypropoxy group, or phenoxy substituted in the para position with a 2-(tetrahydropyran-2-yloxy)ethyl group,

which comprises the following additional steps

a) reacting a compound of formula (1A) or (2A) with the appropriate alcohol derivative to form a compound of formula (3); and

b) optionally converting a compound of formula (3) to a salt.

Another aspect of the invention is the use of a compound of formula (1A) or (2A) or a compound of formula (1) or (2) in the preparation of 1,4-dihydropyridine derivatives of formula (3), especially the stereoisomers thereof.

Another aspect of the invention are 1,4-dihydropyridine derivatives of the formula (3) prepared from a compound of formula (1) or (2).

Another aspect of the invention are 1,4-dihydropyridine derivatives of the formula (3) containing detectable amounts of a resolving agent.

Another aspect of the invention are 1,4-dihydropyridine derivatives of the formula (3) containing detectable amounts of a compound of formula (1) or (2).

Another aspect of the invention is a compound of formula (1), (2), (1A), or (2A) in admixture with a 1,4-dihydro-pyridine derivative of the formula (3).

Another aspect of the invention is a process for preparing a 1,4-dihydropyridine derivative having a carboxylic acid or carboxylic acid derivation function in position 5 of the dihydropyridine ring and a aryl, heterocyclyl, or fused ring heterocycyl, optionally substituted with one, two or three substituents in position 4 of the dihydropyridine ring, or a salt thereof, which process comprises forming a salt mixture of a compound of formula (X)

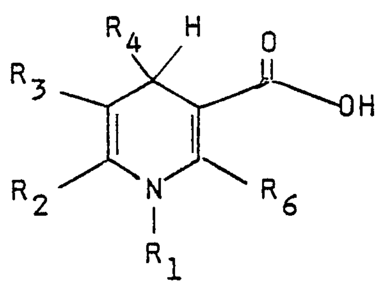

(X)

or a salt thereof

wherein

$R_1$is H or lower alkyl;

$R_2$and $R_6$are each independently lower alkyl, aryl, or arylalkyl;

$R_3$is CN, $NO_2$, $CO_2R_5$, $CONHR_5$, $SO_2R_5$, or $P(O)(OR_5)_2$, where $R_5$is lower alkyl, lower alkoxyalkyl, aryl, or arylalkyl;

$R_4$is aryl, heterocyclyl, or fused-ring heterocyclyl, optionally substituted with one, two, or three substituents independently selected from halo, $NO_2$, CN, lower alkoxy, lower alkylamino, $CF_3$, $OCH_2F$, and $OCF_3$;

with a resolving agent in a solvent selected from dimethyl formamide, ethanol, and acetonitrile, optionally in admixture with water, at a temperature between 50 °C and the reflux temperature of said solvent, optionally adding water during this elevated temperature, such that the ratio of 1,4-dihydropyridine derivative to resolving agent is about 1:2 to about 2:1 and the mixture contains about 10% to about 50% water, wherein said salt mixture comprises

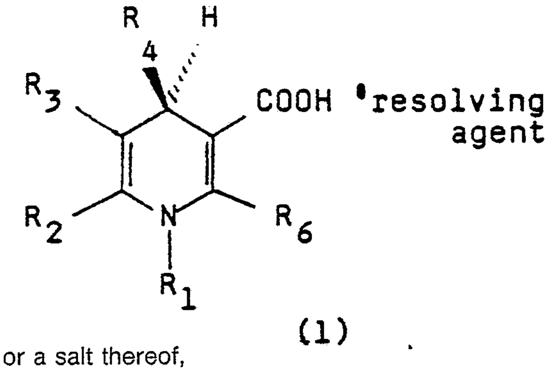

(1)

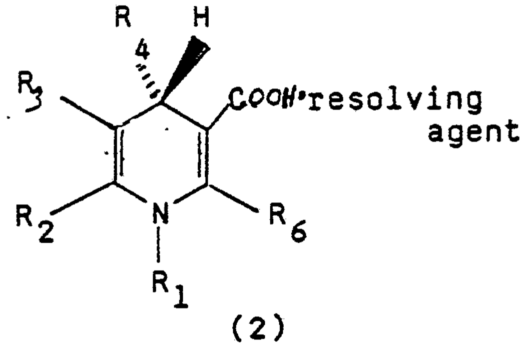

(2)

or a salt thereof,

wherein $R_1$, $R_2$, $R_3$, $R_4$, and $R_6$ are as defined above.

The resolving agents comprise optically active amine bases such as cinchonidine, cinchonine, quinine, quinidine, strychnine, burcine, morphine, L-arginine, D-α-phenylethylamine, dehydroabietylamine, cinchonicine, L-2-amino-1-propanol, D-amphetamine, glucosamine, conessine, anabasine, D-ephedrine, or and N-alkyl-D-glucamine wherein alkyl is C1 to C10.

A preferred class of the invention is the process wherein said resolving agent is cinchonidine, cinchonine or quinidine. A preferred subclass of the invention is the process wherein $R_1$ is H, $R_2$ and $R_6$ are methyl, $R_3$ is CN or $CO_2R_5$ (where $R_5$ is methyl, ethyl, isopropyl, or methoxyethyl), and $R_4$ is phenyl or 2-thienyl, optionally substituted with one, two, or three substituents independently selected from halo, $NO_2$, CN, lower alkyl, lower alkoxy, lower alkylamino, $CF_3$, $OCH_2F$, and $OCF_3$, particularly the process wherein $R_3$ is carbomethoxy and $R_4$ is 3-nitrophenyl.

Another aspect of the invention is a process for the preparation of 1,4-dihydropyridine derivatives, which process further comprises:

a) separating the compound represented by the formula (1) from the compound represented by the formula (2); and

b) optionally purifying the isolated compound of formula (1) or (2).

It should be noted that pyrifying or separating refers to recrystallization or another technique known in the art, for example, filtration, extraction, crystallization, column chromatography, thin layer chromatography, thick layer chromatography, preparative low or high pressure liquid chromatography, distillation, or a combination of these procedures.

Another aspect of the invention is the compound of formula (1) or (2)

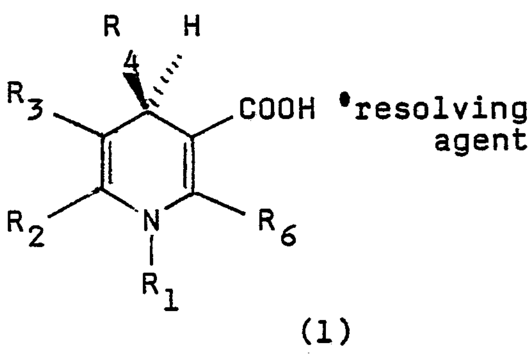

(1)

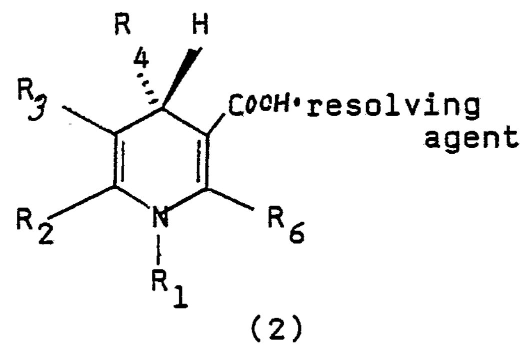

(2)

or a salt thereof

wherein

$R_1$ is H or lower alkyl;

$R_2$ and $R_6$ are each independently lower alkyl, aryl, or arylalkyl;

$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl;

$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$; and

resolving agent comprises an optically active amine base such as cinchonidine, cinchonine, quinine, quinidine, strychnine, brucine, morphine, L-arginine, D-α-phenylethylamine, dehydroabietylamine, conessine, cinchonicine, L-2-amino-1-propanol, D-amphetamine, glucosamine, anabasine, D-ephedrine, or an N-alkyl-D-glucamine wherein alkyl is C1 to C10.

A preferred class of the invention is the compound of formula (1) or (2) wherein said resolving agent is cinchonidine, cinchonine or quinidine. A preferred subclass of the invention is the compound of formula (1) or (2) wherein $R_1$ is H, $R_2$ and $R_6$ are methyl, $R_3$ is cyano, carbomethoxy or carboethoxy, and $R_4$ is 3-nitrophenyl, 2-nitrophenyl, 2-chlorophenyl, or 2,3-dichlorophenyl, particularly the compound of formula (1) or (2) wherein $R_3$ is carbomethoxy and $R_4$ is 3-nitrophenyl.

## DEFINITIONS

As used in the specification and appended claims, unless specified to the contrary, the following terms have the meaning indicated:

The term "lower alkyl" refers to a straight or branched chain monovalent radical consisting solely of carbon and hydrogen, containing no unsaturation and having from one to four carbon atoms. Examples of lower alkyl groups are methyl, ethyl, n-propyl, i-propyl, n-butyl, i-butyl, s-butyl and t-butyl.

The term "alkylene" refers to a divalent radical consisting solely of carbon and hydrogen in the form of $-(CH_2)_n$-wherein n is 1 to 6. Examples of alkylene groups are methylene, ethylene, propylene, butylene, pentylene, and hexylene.

The term "alkoxy" refers to a radical of the form $R_aO$-, where $R_a$ is lower alkyl as defined above.

The term "alkoxyalkyl" refers to radicals of the form $-R_b-O-R_a$, where $R_b$ is alkylene of one to six carbon atoms, and $R_a$ is lower alkyl as defined above. Examples of alkoxyalkyl groups are methoxymethyl, methoxyethyl, 2-(2-propoxy)ethyl, t-butoxymethyl, and the like.

The term "halo" as used herein refers to fluoro, chloro, bromo and iodo.

The term "aryl" refers to a substituted or unsubstituted monovalent unsaturated aromatic carbocyclic radical having a single ring, for example, phenyl, or 2 or 3 condensed rings, for example, β-naphthyl and 3-acenaphthylenyl, having a total of at most 12 ring members; each aromatic ring having from 5 to 7 ring members; phenyl is preferred.

The term "arylalkyl" refers to an aryl group as defined above, attached to an alkyl group wherein alkyl is defined above, for example, phenethyl, phenylpropyl, or benzyl.

The term "heterocyclyl" as used herein refers to saturated and unsaturated cyclic radicals composed of carbon and hydrogen, with oxygen, nitrogen, and/or sulfur heteroatoms. Heterocyclyl groups as referred to herein are rings of 5 or 6 members. Examples of heterocyclyl groups are 2-thienyl, 2-thiazolyl, 2-furyl, 2-pyridyl, and 3-pyridyl.

Fused-ring heterocyclyl groups are benzo-fused heterocyclyl radicals, such as quinolinyl, benzodioxazolyl, benzo[b]thienyl, benzoxazolyl, benzo[b]furanyl, indolyl, and the like. Fused-ring heterocyclyl radicals may be joined to the dihydropyridine ring through any position.

The term "optionally substituted" includes the cases wherein a group is substituted or unsubstituted. As used herein, "optionally substituted" heterocyclyl groups may be substituted with zero, one or two lower alkyl, lower alkoxy, $NO_2$, CN, $CF_3$, $OCF_3$, $OCH_2F$, alkylamino, and dialkylamino, as those terms are defined herein. Thus, the term "optionally substituted heterocyclyl radical" includes, for example, 2-methyltetrahydrofuran-4-yl, 2-methoxytetrahydropyran-4-yl, 2-methylfuran-4-yl, and the like.

The term "salt includes both acidic salts and basic salts.

Acidic salts are those salts which contain inorganic or organic anions, for example, chloride, bromide, iodide, hydroxide, carbonate, bicarbonate, sulfate, nitrate, acetate, benzoate, tosylate, lactate, and the like, for example, HCl.

Basic salts are those salts which contain organic or inorganic cations, for example, aluminum, calcium, lithium, magnesium, potassium, sodium, zinc, barium, bismuth, benzathine, chloroprocaine, choline, diethanolamine, ethylenediamine, meglumine, procaine, tromethamine, and the like.

The term "resolving agent" refers to optically active amine bases such as cinchonidine, quinidine, strychnine, brucine, morphine, cinchonine, quinine, D-α-phenylethylamine, L-arginine, dehydroabietylamine, cinchonicine, L-2-amino-1-propanol, D-amphetamine, glucosamine, conessine, anabasine, D-ephedrine, or an N-alkyl-D-glucamine, wherein alkyl is C1 to C10, for example, N-methyl-D-glucamine, N-ethyl-D-glucamine, N-propyl-D-glucamine, N-butyl-D-glucamine, N-pentyl-D-glucamine, N-hexyl-D-glucamine, N-heptyl-D-glucamine, N-octyl-D-glucamine, N-nonyl-D-glucamine, or N-decyl-D-glucamine.

The term "resolving agent" refers to a suitable optically active amine base having a chiral center which forms an insoluble salt with either the (S) isomer or the (R) isomer of a compound of formula (X) in the solvent selected. Presently preferred resolving agents are cinchonidine, cinchonine, quinidine, and N-alkyl-D-glucamines, where alkyl includes C1 through C10. The presently most preferred resolving agents are

cinchonidine, cinchonine and quinidine.

The term "resolution" or "resolving" refers to a separation of a compound into its enantiomers or a purification of the compound.

The term "purification" or "purifying" refers to recrystallization or use of another technique known in the art, for example, filtration, extraction, crystallization, column chromatography, thin layer chromatography, thick layer chromatography, preparative low or high pressure liquid chromatography, distillation, resolution using resolving agent followed by resolution with another resolving agent wherein one or both of the resolving agents may not be one of the resolving agents defined herein, or a combination of these procedures.

The term "compound" as used, for example, in "compound of formula (X)", refers to either the racemic or non-racemic forms of the compound. Racemic or non-racemic refers to mixtures of the enantiomers of a chiral molecule wherein the ratio of the amount of one enantiomer to the amount of its optical isomer is 1:1 or is not 1:1, respectively.

The term "optically pure" refers to the presence of a significant amount of one isomer over another isomer. This term includes not only 100% of one isomer, but also compounds which have small amounts of another isomer present.

The term "cleaving" refers to the removal of one chemical group from another chemical group, for example, the removal of a salt to form the free compound.

The term "mineral acid" refers to protic acids which have a $pK_a$ lower than 5. Examples of mineral acids include HCl, HBr, $H_2SO_4$, $HNO_3$, and the like.

The term "soft acid" refers to protic organic acids which have a $pK_a$ higher than mineral acids. Examples of soft acids include acetic acid, oxalic acid, toluic acid, and the like.

The nomenclature used herein is a modified form of the I.U.P.A.C. convention. Compounds of the invention are named as derivatives of 1,4-dihydropyridine. The positions in the compounds are numbered beginning with the pyridine nitrogen and proceeding clockwise in all drawings of the structure. For example, the following compound is named 2,6-dimethyl-3-carbomethoxy-4-(3-nitrophenyl)-5-carboxy-1,4-dihydropyridine:

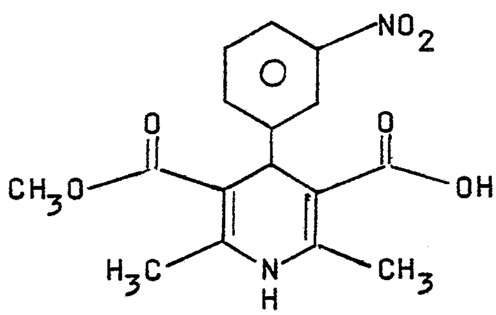

It should be noted that using other nomenclature the 1,4-dihydropyridine derivatives of this invention may be named proceeding counterclockwise, which would result in the carboxylic acid function being labelled as the 3-position. It is intended that the compounds of this invention have the basic structure shown in formula (X).

Compounds of formula (X) have a chiral center at C4 in the dihydropyridine ring, and thus can exist as optical isomers. Optical isomers of compounds may be specified (+) or (-), indicating the direction the chiral center rotates a plane of polarized light.

Optically active intermediates and compounds of formula (X) may also be designated using the IUPAC R-S convention, sometimes called the "sequence rule." A description of the R-S convention may be found, for example, in "Introduction to Organic Chemistry" by A. Streitwieser, Jr. and C. Heathcock, (Macmillan Pub. Co., New York, 1976), pages 110-114. For example, the compound depicted below is (S)-2,6-dimethyl-3-(2-propoxycarbonyl)-4-(3-nitrophenyl)-5-carboxy-1,4-dihydropyridine:

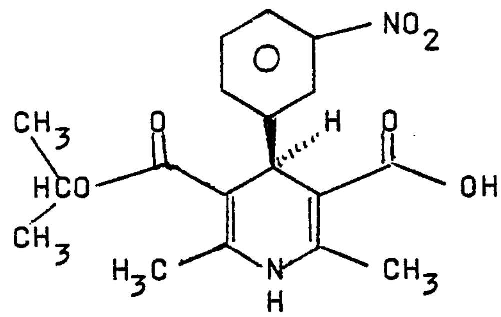

## PREPARATION OF THE INVENTION

Compounds and salts of formula (1A) or (2A) are prepared by following the method of the invention. A compound of formula (X) is first obtained using methods known in the art. For example, one may use the Hantzsch Dihydropyridine synthesis, using reactants selected to provide the desired substitutions for $R_1$, $R_2$, $R_3$, and $R_4$. Compounds wherein $R_3$ is $CO_2R_5$, $NO_2$, CN, or $CONHR_5$ can be prepared by reacting an aryl aldehyde, acetoacetic acid, and a compound of the formula $R_3$-CH=C($NH_2$)$R_2$ under Hantzsch conditions (e.g., refluxing EtOH) to provide an unresolved dihydropyridine derivative. For example, methyl $\beta$-aminocrotonate, 3-nitrobenzaldehyde and acetoacetic acid are heated at reflux in ethanol to yield unresolved 2,6-dimethyl-3-methoxycarbonyl-4-(3-nitrophenyl)-5-carboxy-1,4-dihydropyridine. Other compounds of formula (X) may similarly be prepared by following the teachings of U.S. Pat. No. 4,595,690, incorporated herein by reference in its entirety.

Compounds of formula (X) in which $R_3$ is $SO_2R_5$ may be similarly prepared, or may be prepared by following the methods taught in U.S. Pat. No. 4,126,321, incorporated herein by reference in full.

Compounds of formula (X) in which $R_3$ is P(O)($OR_5$)$_2$ may similarly be prepared, or may be prepared by following the methods taught in U.S. Pat. Nos. 4,576,934 and 4,535,073, incorporated herein by reference in full.

Once the compound of formula (X) has been obtained, it is resolved into optically pure isomers by following the method of the instant invention. This process is a process for preparing a 1,4-dihydropyridine derivative having a carboxylic acid or carboxylic acid derivative function in position 5 of the dihydropyridine ring and an aryl, heterocyclyl, or fused ring heterocycyl, optionally substituted with one, two or three substituents in postion 4 of the dihydropyridine ring, or a salt thereof, which process comprises forming a salt mixture of a compound of formula (X)

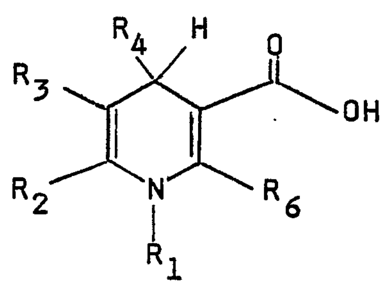

(X)

or a salt thereof

wherein

$R_1$ is H or lower alkyl;

$R_2$ and $R_6$ are each independently lower alkyl, aryl, or arylalkyl;

$R_3$ is CN, $NO_2$, $CO_2R_5$, $CONHR_5$, $SO_2R_5$, or P(O)($OR_5$)$_2$, where $R_5$ is lower alkyl, lower alkoxyalkyl, aryl, arylalkyl;

$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$;

with a resolving agent in a solvent selected from dimethyl formamide, ethanol, and acetonitrile, optionally in

admixture with water, at a temperature between 50°C and the reflux temperature of said solvent, optionally adding water during this elevated temperature, such that the ratio of 1,4-dihydropyridine derivative to resolving agent is about 1:2 to about 2:1 and the mixture contains about 10% to about 50% water, wherein said salt mixture comprises

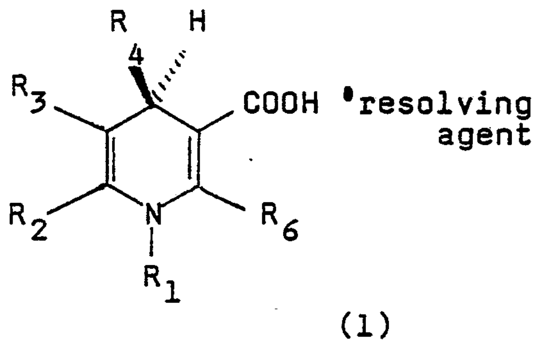
(1)

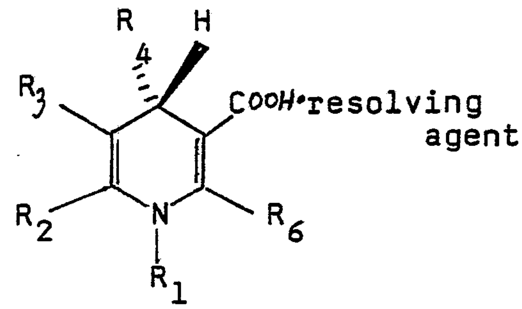
(2)

or a salt thereof
wherein $R_1$, $R_2$, $R_3$, $R_4$, and $R_6$ are as defined above.

The preferred amount of water depends on which solvent is selected. For DMF, the preferred percentage of water is 30% to 50%, particularly about 40%. For $CH_3CN$, the preferred percentage of water is about 20% to 40%, particularly about 20%. For EtOH, the preferred percentage of water is about 10% to 30%, particularly about 20%. Where the solvent is dimethyl formamide, the water may optionally be added at the next step. When the amount of resolving agent used is less than the amount of dihydropyridine, an inexpensive base such as NaOH or $NH_4OH$ may be used to make up the difference. The resolving agent is, for example, cinchonidine, quinidine, strychnine, brucine, morphine, cinchonine, quinine, D-α-phenylethylamine, L-arginine, conessine, dehydroabietylamine, cinchonicine, L-2-amino-1-propanol, D-amphetamine, glucosamine, anabasine, D-ephedrine, or an N-alkyl-D-glucamine, wherein alkyl is C1 to C10, with cinchonidine and quinidine being the most preferred.

At this point, the mixture is allowed to stand and cool gradually, so that the a compound of formula (1) or (2) forms. A compound of formula (1) or (2) can be separated and/or purified by any of a number of procedures known in the art, comprising fractional crystallization, thin layer chromatography, thick layer chromatography, column chromatography, preparative low or high pressure liquid chromatography, or resolution using a resolving agent followed by resolution with another resolving agent. Presently preferred is fractional crystallization wherein one compound of formula (1) or (2) crystallizes from the resolution mixture. The crystalline compound of formula (1) or (2) is filtered, washed, and dried to yield an optically pure dihydropyridine derivative of formula (1) or (2), and the resulting compound of formula (1) or (2) can be recrystallized, e.g. from EtOH. The pure optically active compound of formula (1) or (2) may then be cleaved by dissolving it in a strong acid, for example, HCl, $H_2SO_4$, or the like, or in a strong base, for example, NaOH, $NH_4OH$, or the like, followed by a strong acid to yield a pure optically active dihydropyridine acid of formula (1A) or (2A). The other optical isomer may similarly be recovered from the resolution solution. The undesired optical isomer may also be racemized and recycled or resolved to form more of the desired optical isomer. This racemization and recyclization may be performed according to the resolution process of this case, or by another resolution process known in the art.

The resulting optically pure dihydropyridine acid derivatives may be esterified or amidated by means known in the art to produce pharmaceutically active agents which modulate calcium channel activity, and generally affect cerebrovascular and cardiovascular activity. For example, (-)-2,6-dimethyl-3-carbomethoxy-4-(3-nitrophenyl)-5-carboxy-1,4-dihydropyridine may be esterified with 2-(N-methyl-N-benzylamino)ethanol to yield the (+) optical isomer of 2,-dimethyl-3-carboxymethoxy-4-(3-nitrophenyl)-5-[2-(N-methyl-N-benzylamino)ethoxycarbonyl]-1,4-dihydropyridine, (also known as nicardapine) having the greatest biological activity. Similarly, (-)-2,6-dimethyl-3-carbomethoxy-4-(3-nitrophenyl)-5-carboxy-1,4-dihydropyridine may be esterified with ethanol to yield the optical isomer of 2,6-dimethyl-3-carbomethoxy-4-(3-nitrophenyl)-5-carboethoxy-1,4-dihydropyridine, (also known as nitrendipine) having the greatest biological activity.

It is well known that pharmaceuticals must meet pharmacopoeia standards before approval and/or marketing. Resolving agents and/or their impurities such as intermediates, reagents, solvents, etc., should not exceed the limits prescribed by pharmacopoeia standards. 1,4-Dihydropyridine derivative pharmaceuticals prepared using a resolving agent inevitably will have minor amounts of the resolving agent present. It is important to monitor 1,4-dihydropyridine pharmaceuticals for the presence of such resolving agents.

The following examples are presented as further illustrations of the practice and preparation of the invention, and are not intended as limitations on the scope of the invention.

EXAMPLE 1

(Preparation of Quinidine Salts)

(A) A mixture of 2,6-dimethyl-3-carbomethoxy-4-(3-nitrophenyl)-5-carboxy-1,4-dihydropyridine (40.0 g, 0.12 mol) and anhydrous quinidine (39 g, 0.12 mol) was dissolved in hot (about 90°C) DMF (72 mL). Then, water (48 mL) was added and the solution allowed to cool to room temperature. After 24 hours, the resulting precipitate was filtered, dried, and recrystallized from hot DMF/$H_2O$ (60:40) t afford the quinidine salt of 2,6-dimethyl-3-carbomethoxy-4-(3-nitrophenyl)-5-carboxy-1,4-dihydropyridine (mp = 165°C).

The pure (-) 2,6-dimethyl-3-carbomethoxy-4-(3-nitrophenyl)-5-carboxy-1,4-dihydropyridine was obtained by dissolving 17.8 g of the quinidinium salt and NaOH (2.7 mL of 35% soln) in water (60 mL). The solution was extracted twice with $CH_2Cl_2$ (30 mL), then acidified with HCl (12N, 3.4 mL). The resulting precipitate was filtered, washed with $H_2O$, and dried. The dried product was rinsed with $Et_2O$ (2 x 50 mL) to yield pure (-)-2,6-dimethyl-3-carbomethoxy-4-(3-nitrophenyl)-5-carboxy-1,4-dihydropyridine, $[\alpha]_D^{20}$ = -20.4° (c=0.5, acetone), mp = 200°C.

(B) Similarly, proceeding as in part (A) above but substituting the appropriate starting materials for 2,6-dimethyl-3-carbomethoxy-4-(3-nitrophenyl)-5-carboxy-1,4-dihydropyridine, the following optically resolved compounds are prepared:

2,6-dimethyl-3-carbomethoxy-4-(2-nitrophenyl)-5-carboxy-1,4-dihydropyridine;
2,6-dimethyl-3-carbomethoxy-4-(2-chlorophenyl)-5-carboxy-1,4-dihydropyridine;
(-)-2,6-dimethyl-3-carbomethoxy-4-(3-chlorophenyl)-5-carboxy-1,4-dihydropyridine, mp = 210°C, $[\alpha]_D^{20}$ = -8.0°, (c = 5, DMF);
2,6-dimethyl-3-carbomethoxy-4-(2,3-dichlorophenyl)-5-carboxy-1,4-dihydropyridine;
2,6-dimethyl-3-carbomethoxy-4-(4-methylphenyl)-5-carboxy-1,4-dihydropyridine;
2,6-dimethyl-3-carbomethoxy-4-(3-cyanophenyl)-5-carboxy-1,4-dihydropyridine;
2,6-dimethyl-3-carbomethoxy-4-(3-trifluoromethylphenyl)-5-carboxy-1,4-dihydropyridine;
2,6-dimethyl-3-carbomethoxy-4-(3-trifluoromethoxyphenyl)-5-carboxy-1,4-dihydropyridine;
(+)-2,6-dimethyl-3-carbomethoxy-4-(3-methoxyphenyl)-5-carboxy-1,4-dihydropyridine, mp = 235°C, $[\alpha]_D^{20}$ = +1.7°, (c = 5, DMF);
(+)-2,6-dimethyl-3-carbomethoxy-4-(2-thienyl)-5-carboxy-1,4-dihydropyridine, mp = 200°C, $[\alpha]_D^{20}$ = +6.5°, (c = 5, DMF);
2,6-dimethyl-3-carbomethoxy-4-(5-methylthien-2-yl)-5-carboxy-1,4-dihydropyridine;
2,6-dimethyl-3-carboethoxy-4-(2-nitrophenyl)-5-carboxy-1,4-dihydropyridine;
2,6-dimethyl-3-carboethoxy-4-(3-nitrophenyl)-5-carboxy-1,4-dihydropyridine;
2,6-dimethyl-3-carboethoxy-4-(3-chlorophenyl)-5-carboxy-1,4-dihydropyridine;
2,6-dimethyl-3-carboethoxy-4-(2-chlorophenyl)-5-carboxy-1,4-dihydropyridine;
2,6-dimethyl-3-carboethoxy-4-(2,3-dichlorophenyl)-5-carboxy-1,4-dihydropyridine;
2,6-dimethyl-3-carboethoxy-4-(4-methylphenyl)-5-carboxy-1,4-dihydropyridine;
2,6-dimethyl-3-carboethoxy-4-(3-cyanophenyl)-5-carboxy-1,4-dihydropyridine;
2,6-dimethyl-3-carboethoxy-4-(3-trifluoromethylphenyl)-5-carboxy-1,4-dihydropyridine;
2,6-dimethyl-3-carboethoxy-4-(3-trifluoromethoxyphenyl)-5-carboxy-1,4-dihydropyridine;
2,6-dimethyl-3-carboisopropoxy-4-(2-nitrophenyl)-5-carboxy-1,4-dihydropyridine;
2,6-dimethyl-3-carboisopropoxy-4-(3-nitrophenyl)-5-carboxy-1,4-dihydropyridine;
2,6-dimethyl-3-carboisopropoxy-4-(3-chlorophenyl)-5-carboxy-1,4-dihydropyridine;
2,6-dimethyl-3-carboisopropoxy-4-(2-chlorophenyl)-5-carboxy-1,4-dihydropyridine;
2,6-dimethyl-3-carboisopropoxy-4-(2,3-dichlorophenyl)-5-carboxy-1,4-dihydropyridine;
2,6-dimethyl-3-carboisopropoxy-4-(4-methylphenyl)-5-carboxy-1,4-dihydropyridine;
2,6-dimethyl-3-carboisopropoxy-4-(3-cyanophenyl)-5-carboxy-1,4-dihydropyridine;
2,6-dimethyl-3-carboisopropoxy-4-(3-trifluoromethylphenyl)-5-carboxy-1,4-dihydropyridine;
2,6-dimethyl-3-carboisopropoxy-4-(3-trifluoromethoxyphenyl)-5-carboxy-1,4-dihydropyridine;
2,6-dimethyl-3-methylsulfonyl-4-(2-nitrophenyl)-5-carboxy-1,4-dihydropyridine;
2,6-dimethyl-3-methylsulfonyl-4-(3-nitrophenyl)-5-carboxy-1,4-dihydropyridine;
2,6-dimethyl-3-methylsulfonyl-4-(3-chlorophenyl)-5-carboxy-1,4-dihydropyridine;
2,6-dimethyl-3-methylsulfonyl-4-(2-chlorophenyl)-5-carboxy-1,4-dihydropyridine;

2,6-dimethyl-3-methylsulfonyl-4-(2,3-dichlorophenyl)-5-carboxy-1,4-dihydropyridine;
2,6-dimethyl-3-methylsulfonyl-4-(4-methylphenyl)-5-carboxy-1,4-dihydropyridine;
2,6-dimethyl-3-methylsulfonyl-4-(3-cyanophenyl)-5-carboxy-1,4-dihydropyridine;
2,6-dimethyl-3-methylsulfonyl-4-(3-trifluoromethylphenyl)-5-carboxy-1,4-dihydropyridine;
2,6-dimethyl-3-methylsulfonyl-4-(3-trifluoromethoxyphenyl)-5-carboxy-1,4-dihydropyridine;
2,6-dimethyl-3-ethylsulfonyl-4-(2-nitrophenyl)-5-carboxy-1,4-dihydropyridine;
2,6-dimethyl-3-ethylsulfonyl-4-(3-nitrophenyl)-5-carboxy-1,4-dihydropyridine;
2,6-dimethyl-3-ethylsulfonyl-4-(3-chlorophenyl)-5-carboxy-1,4-dihydropyridine;
2,6-dimethyl-3-ethylsulfonyl-4-(2-chlorophenyl)-5-carboxy-1,4-dihydropyridine;
2,6-dimethyl-3-ethylsulfonyl-4-(2,3-dichlorophenyl)-5-carboxy-1,4-dihydropyridine;
2,6-dimethyl-3-ethylsulfonyl-4-(4-methylphenyl)-5-carboxy-1,4-dihydropyridine;
2,6-dimethyl-3-ethylsulfonyl-4-(3-cyanophenyl)-5-carboxy-1,4-dihydropyridine;
2,6-dimethyl-3-ethylsulfonyl-4-(3-trifluoromethylphenyl)-5-carboxy-1,4-dihydropyridine;
2,6-dimethyl-3-ethylsulfonyl-4-(3-trifluoromethoxyphenyl)-5-carboxy-1,4-dihydropyridine;
2,6-dimethyl-3-isopropylsulfonyl-4-(2-nitrophenyl)-5-carboxy-1,4-dihydropyridine;
2,6-dimethyl-3-isopropylsulfonyl-4-(3-nitrophenyl)-5-carboxy-1,4-dihydropyridine;
2,6-dimethyl-3-isopropylsulfonyl-4-(3-chlorophenyl)-5-carboxy-1,4-dihydropyridine;
2,6-dimethyl-3-isopropylsulfonyl-4-(2-chlorophenyl)-5-carboxy-1,4-dihydropyridine;
2,6-dimethyl-3-isopropylsulfonyl-4-(2,3-dichlorophenyl)-5-carboxy-1,4-dihydropyridine;
2,6-dimethyl-3-isopropylsulfonyl-4-(4-methylphenyl)-5-carboxy-1,4-dihydropyridine;
2,6-dimethyl-3-isopropylsulfonyl-4-(3-cyanophenyl)-5-carboxy-1,4-dihydropyridine;
2,6-dimethyl-3-isopropylsulfonyl-4-(3-trifluoromethylphenyl)-5-carboxy-1,4-dihydropyridine;
2,6-dimethyl-3-isopropylsulfonyl-4-(3-trifluoromethoxyphenyl)-5-carboxy-1,4-dihydropyridine;
2,6-dimethyl-3-dimethylphosphonyl-4-(2-nitrophenyl)-5-carboxy-1,4-dihydropyridine;
2,6-dimethyl-3-dimethylphosphonyl-4-(3-nitrophenyl)-5-carboxy-1,4-dihydropyridine;
2,6-dimethyl-3-dimethylphosphonyl-4-(3-chlorophenyl)-5-carboxy-1,4-dihydropyridine;
2,6-dimethyl-3-dimethylphosphonyl-4-(2-chlorophenyl)-5-carboxy-1,4-dihydropyridine;
2,6-dimethyl-3-dimethylphosphonyl-4-(2,3-dichlorophenyl)-5-carboxy-1,4-dihydropyridine;
2,6-dimethyl-3-dimethylphosphonyl-4-(4-methylphenyl)-5-carboxy-1,4-dihydropyridine;
2,6-dimethyl-3-dimethylphosphonyl-4-(3-cyanophenyl)-5-carboxy-1,4-dihydropyridine;
2,6-dimethyl-3-dimethylphosphonyl-4-(3-trifluoromethylphenyl)-5-carboxy-1,4-dihydropyridine;
2,6-dimethyl-3-dimethylphosphonyl-4-(3-trifluoromethoxyphenyl)-5-carboxy-1,4-dihydropyridine;
2,6-dimethyl-3-diethylphosphonyl-4-(2-nitrophenyl)-5-carboxy-1,4-dihydropyridine;
2,6-dimethyl-3-diethylphosphonyl-4-(3-nitrophenyl)-5-carboxy-1,4-dihydropyridine;
2,6-dimethyl-3-diethylphosphonyl-4-(3-chlorophenyl)-5-carboxy-1,4-dihydropyridine;
2,6-dimethyl-3-diethylphosphonyl-4-(2-chlorophenyl)-5-carboxy-1,4-dihydropyridine;
2,6-dimethyl-3-diethylphosphonyl-4-(2,3-dichlorophenyl)-5-carboxy-1,4-dihydropyridine;
2,6-dimethyl-3-diethylphosphonyl-4-(4-methylphenyl)-5-carboxy-1,4-dihydropyridine;
2,6-dimethyl-3-diethylphosphonyl-4-(3-cyanophenyl)-5-carboxy-1,4-dihydropyridine;
2,6-dimethyl-3-diethylphosphonyl-4-(3-trifluoromethylphenyl)-5-carboxy-1,4-dihydropyridine;
2,6-dimethyl-3-diethylphosphonyl-4-(3-trifluoromethoxyphenyl)-5-carboxy-1,4-dihydropyridine;
2,6-dimethyl-3-diisopropylphosphonyl-4-(2-nitrophenyl)-5-carboxy-1,4-dihydropyridine;
2,6-dimethyl-3-diisopropylphosphonyl-4-(3-nitrophenyl)-5-carboxy-1,4-dihydropyridine;
2,6-dimethyl-3-diisopropylphosphonyl-4-(3-chlorophenyl)-5-carboxy-1,4-dihydropyridine;
2,6-dimethyl-3-diisopropylphosphonyl-4-(2-chlorophenyl)-5-carboxy-1,4-dihydropyridine;
2,6-dimethyl-3-diisopropylphosphonyl-4-(2,3-dichlorophenyl)-5-carboxy-1,4-dihydropyridine;
2,6-dimethyl-3-diisopropylphosphonyl-4-(4-methylphenyl)-5-carboxy-1,4-dihydropyridine;
2,6-dimethyl-3-diisopropylphosphonyl-4-(3-cyanophenyl)-5-carboxy-1,4-dihydropyridine;
2,6-dimethyl-3-diisopropylphosphonyl-4-(3-trifluoromethylphenyl)-5-carboxy-1,4-dihydropyridine;
2,6-dimethyl-3-diisopropylphosphonyl-4-(3-trifluoromethoxyphenyl)-5-carboxy-1,4-dihydropyridine;
2,6-dimethyl-3-methoxyethoxycarbonyl-4-(2-nitrophenyl)-5-carboxy-1,4-dihydropyridine;
2,6-dimethyl-3-methoxyethoxycarbonyl-4-(3-chlorophenyl)-5-carboxy-1,4-dihydropyridine;
2,6-dimethyl-3-methoxyethoxycarbonyl-4-(2-chlorophenyl)-5-carboxy-1,4-dihydropyridine;
2,6-dimethyl-3-methoxyethoxycarbonyl-4-(2,3-dichlorophenyl)-5-carboxy-1,4-dihydropyridine;
2,6-dimethyl-3-methoxyethoxycarbonyl-4-(4-methylphenyl)-5-carboxy-1,4-dihydropyridine;
2,6-dimethyl-3-methoxyethoxycarbonyl-4-(3-cyanophenyl)-5-carboxy-1,4-dihydropyridine;
2,6-dimethyl-3-methoxyethoxycarbonyl-4-(3-trifluoromethylphenyl)-5-carboxy-1,4-dihydropyridine;
2,6-dimethyl-3-methoxyethoxycarbonyl-4-(3-trifluoromethoxyphenyl)-5-carboxy-1,4-dihydropyridine;

2,6-dimethyl-3-methoxyethoxycarbonyl-4-(3-methoxyphenyl)-5-carboxy-1,4-dihydropyridine;
2,6-dimethyl-3-methoxyethoxycarbonyl-4-(2-thienyl)-5-carboxy-1,4-dihydropyridine;
2,6-dimethyl-3-methoxyethoxycarbonyl-4-(5-methylthien-2-yl)-5-carboxy-1,4-dihydropyridine;
2,6-dimethyl-3-cyano-4-(2-nitrophenyl)-5-carboxy-1,4-dihydropyridine;
(+)-2,6-dimethyl-3-cyano-4-(3-nitrophenyl)-5-carboxy-1,4-dihydropyridine, mp = 225°C, $[\alpha]_D^{20}$ = +251.2°, (c = 5, DMF);
2,6-dimethyl-3-cyano-4-(3-chlorophenyl)-5-carboxy-1,4-dihydropyridine;
2,6-dimethyl-3-cyano-4-(2-chlorophenyl)-5-carboxy-1,4-dihydropyridine;
2,6-dimethyl-3-cyano-4-(2,3-dichlorophenyl)-5-carboxy-1,4-dihydropyridine;
2,6-dimethyl-3-cyano-4-4-methylphenyl)-5-carboxy-1,4-dihydropyridine;
2,6-dimethyl-3-cyano-4-(3-cyanophenyl)-5-carboxy-1,4-dihydropyridine;
2,6-dimethyl-3-cyano-4-(3-trifluoromethylphenyl)-5-carboxy-1,4-dihydropyridine;
2,6-dimethyl-3-cyano-4-(3-trifluoromethoxyphenyl)-5-carboxy-1,4-dihydropyridine;
2,6-dimethyl-3-cyano-4-(3-methoxyphenyl)-5-carboxy-1,4-dihydropyridine;
2,6-dimethyl-3-cyano-4-(2-thienyl)-5-carboxy-1,4-dihydropyridine;
2,6-dimethyl-3-cyano-4-(5-methylthien-2-yl)-5-carboxy-1,4-dihydropyridine;
2,6-dimethyl-3-nitro-4-(2-nitrophenyl)-5-carboxy-1,4-dihydropyridine;
2,6-dimethyl-3-nitro-4-(3-nitrophenyl)-5-carboxy-1,4-dihydropyridine;
2,6-dimethyl-3-nitro-4-(3-chlorophenyl)-5-carboxy-1,4-dihydropyridine;
2,6-dimethyl-3-nitro-4-(2-chlorophenyl)-5-carboxy-1,4-dihydropyridine;
2,6-dimethyl-3-nitro-4-(2,3-dichlorophenyl)-5-carboxy-1,4-dihydropyridine;
2,6-dimethyl-3-nitro-4-(4-methylphenyl)-5-carboxy-1,4-dihydropyridine;
2,6-dimethyl-3-nitro-4-(3-nitrophenyl)-5-carboxy-1,4-dihydropyridine;
2,6-dimethyl-3-nitro-4-(3-trifluoromethylphenyl)-5-carboxy-1,4-dihydropyridine;
2,6-dimethyl-3-nitro-4-(3-trifluoromethoxyphenyl)-5-carboxy-1,4-dihydropyridine;
2,6-dimethyl-3-nitro-4-(3-methoxyphenyl)-5-carboxy-1,4-dihydropyridine;
2,6-dimethyl-3-nitro-4-(2-thienyl)-5-carboxy-1,4-dihydropyridine;
2,6-dimethyl-3-nitro-4-(5-methyl-2-thienyl)-5-carboxy-1,4-dihydropyridine;
2,6-dimethyl-3-carbomethoxy-4-(2-furanyl)-5-carboxy-1,4-dihydropyridine;
2,6-dimethyl-3-carboethoxy-4-(2-furanyl)-5-carboxy-1,4-dihydropyridine;
2,6-dimethyl-3-carboisopropoxy-4-(2-furanyl)-5-carboxy-1,4-dihydropyridine;
2,6-dimethyl-3-carboisobutoxy-4-(2-furanyl)-5-carboxy-1,4-dihydropyridine;
2,6-dimethyl-3-methylsulfonyl-4-(2-furanyl)-5-carboxy-1,4-dihydropyridine;
2,6-dimethyl-3-cyano-4-(2-furanyl)-5-carboxy-1,4-dihydropyridine;
2,6-dimethyl-3-nitro-4-(2-furanyl)-5-carboxy-1,4-dihydropyridine;
2,6-dimethyl-3-carbomethoxy-4-(2-pyridyl)-5-carboxy-1,4-dihydropyridine;
2,6-dimethyl-3-carboethoxy-4-(2-pyridyl)-5-carboxy-1,4-dihydropyridine;
2,6-dimethyl-3-carboisopropoxy-4-(2-pyridyl)-5-carboxy-1,4-dihydropyridine;
2,6-dimethyl-3-carboisobutoxy-4-(2-pyridyl)-5-carboxy-1,4-dihydropyridine;
2,6-dimethyl-3-methylsulfonyl-4-(2-pyridyl)-5-carboxy-1,4-dihydropyridine;
2,6-dimethyl-3-cyano-4-(2-pyridyl)-5-carboxy-1,4-dihydropyridine;
2,6-dimethyl-3-nitro-4-(2-pyridyl)-5-carboxy-1,4-dihydropyridine;
2,6-dimethyl-3-carbomethoxy-4-(4-benzodioxazolyl)-5-carboxy-1,4-dihydropyridine;
2,6-dimethyl-3-carboethoxy-4-(4-benzodioxazolyl)-5-carboxy-1,4-dihydropyridine;
2,6-dimethyl-3-carboisopropoxy-4-(4-benzodioxazolyl)-5-carboxy-1,4-dihydropyridine;
2,6-dimethyl-3-carboisopropoxy-4-(4-benzodioxazolyl)-5-carboxy-1,4-dihydropyridine;
2,6-dimethyl-3-methylsulfonyl-4-(4-benzodioxazolyl)-5-carboxy-1,4-dihydropyridine;
2,6-dimethyl-3-cyano-4-(4-benzodioxazolyl)-5-carboxy-1,4-dihydropyridine; and
2,6-dimethyl-3-nitro-4-(4-benzodioxazolyl)-5-carboxy-1,4-dihydropyridine.

(C) The mother liquors obtained in part (A) above are then evaporated, and the residue suspended in $H_2O$ (400 mL) containing NaOH (22 mL of 35% solution). The aqueous phase is then washed with $CH_2Cl_2$ (2 x 200 mL) and acidified with HCl (12N, 22 mL). The precipitate is then filtered, washed with water, and dried to obtain nearly pure (+)-2,6-dimethyl-3-carbomethoxy-4-(3-nitrophenyl)-5-carboxy-1,4-dihydropyridine, mp = 202-203°C.

(D) Similarly, proceeding as in part (C) above but substituting the mother liquors obtained in part (B) above for the mother liquor obtained from part (A), the corresponding compounds are prepared.

(E) Similarly, proceeding as in parts (A-B) but substituting cinchonidine for quinidine, the corresponding pure (+) and (-) isomers are obtained.

EXAMPLE 2

(Preparation of Cinchonidine Salts)

(A) A mixture of 2,6-dimethyl-3-carbomethoxy-4-(3-nitrophenyl)-5-carboxy-1,4-dihydropyridine (66.4 g, 0.2 mol) and cinchonidine (59 g, 0.2 mol) was dissolved in hot (about 90°C) DMF (120 mL). When both compounds has dissolved, $H_2O$ (100 mL) was added and the solution allowed to cool for 24 hours under ambient conditions. The resulting precipitate was filtered, dried, and recrystallized from DMF/$H_2O$ (60:40) to yield the pure salt of 2,6-dimethyl-3-carbomethoxy-4-(3-nitrophenyl)-5-carboxy-1,4-dihydropyridine, mp = 200-205°C. (One may also recrystallize the product from EtOH or MeOH.)

The pure (+) 2,6-dimethyl-3-carbomethoxy-4-(3-nitrophenyl)-5-carboxy-1,4-dihydropyridine was obtained by dissolving 17.8 g of the cinchonidinium salt and NaOH (2.7 mL of 35% soln) in water (60 mL). The solution was extracted twice with $CH_2Cl_2$ (30 mL), then acidified with HCl (12N, 3.4 mL). The resulting precipitate was filtered, washed with $H_2O$, and dried. The dried product was rinsed with $Et_2O$ (2 x 50 mL) to yield pure (+)-2,6-dimethyl-3-carbomethoxy-4-(3-nitro phenyl)-5-carboxy-1,4-dihydropyridine, $[\alpha]_D^{20}$ = +20.3° (c=0.5, acetone), mp = 202-203°C.

(B) Similarly, proceeding as in part (A) above but substituting the appropriate starting materials for 2,6-dimethyl-3-carbomethoxy-4-(3-nitrophenyl)-5-carboxy-1,4-dihydropyridine, the following optically resolved compounds are prepared:

2,6-dimethyl-3-carbomethoxy-4-(2-nitrophenyl)-5-carboxy-1,4-dihydropyridine;
2,6-dimethyl-3-carbomethoxy-4-(3-chlorophenyl)-5-carboxy-1,4-dihydropyridine;
2,6-dimethyl-3-carbomethoxy-4-(2-chlorophenyl)-5-carboxy-1,4-dihydropyridine;
(-)-2,6-dimethyl-3-carbomethoxy-4-(2,3-dichlorophenyl)-5-carboxy-1,4-dihydropyridine, mp = 225°C, $[\alpha]_D^{20}$ = -28.5°, (c = 5; DMF);
2,6-dimethyl-3-carbomethoxy-4-(4-methylphenyl)-5-carboxy-1,4-dihydropyridine;
2,6-dimethyl-3-carbomethoxy-4-(3-cyanophenyl)-5-carboxy-1,4-dihydropyridine;
2,6-dimethyl-3-carbomethoxy-4-(3-trifluoromethylphenyl)-5-carboxy-1,4-dihydropyridine;
2,6-dimethyl-3-carbomethoxy-4-(3-trifluoromethoxyphenyl)-5-carboxy-1,4-dihydropyridine;
2,6-dimethyl-3-carboethoxy-4-(2-nitrophenyl)-5-carboxy-1,4-dihydropyridine;
2,6-dimethyl-3-carboethoxy-4-(3-nitrophenyl)-5-carboxy-1,4-dihydropyridine;
2,6-dimethyl-3-carboethoxy-4-(3-chlorophenyl)-5-carboxy-1,4-dihydropyridine;
2,6-dimethyl-3-carboethoxy-4-(2-chlorophenyl)-5-carboxy-1,4-dihydropyridine;
2,6-dimethyl-3-carboethoxy-4-(2,3-dichlorophenyl)-5-carboxy-1,4-dihydropyridine;
2,6-dimethyl-3-carboethoxy-4-(4-methylphenyl)-5-carboxy-1,4-dihydropyridine;
2,6-dimethyl-3-carboethoxy-4-(3-cyanophenyl)-5-carboxy-1,4-dihydropyridine;
2,6-dimethyl-3-carboethoxy-4-(3-trifluoromethylphenyl)-5-carboxy-1,4-dihydropyridine;
2,6-dimethyl-3-carboethoxy-4-(3-trifluoromethoxyphenyl)-5-carboxy-1,4-dihydropyridine;
2,6-dimethyl-3-carboisopropoxy-4-(2-nitrophenyl)-5-carboxy-1,4-dihydropyridine;
(-)-2,6-dimethyl-3-carboisopropoxy-4-(3-nitrophenyl)-5-carboxy-1,4-dihydropyridine, mp = 195°C, $[\alpha]_D^{20}$ = -28.9°, (c = 5, DMF);
2,6-dimethyl-3-carboisopropoxy-4-(3-chlorophenyl)-5-carboxy-1,4-dihydropyridine;
2,6-dimethyl-3-carboisopropoxy-4-(2-chlorophenyl)-5-carboxy-1,4-dihydropyridine;
2,6-dimethyl-3-carboisopropoxy-4-(2,3-dichlorophenyl)-5-carboxy-1,4-dihydropyridine;
2,6-dimethyl-3-carboisopropoxy-4-(4-methylphenyl)-5-carboxy-1,4-dihydropyridine;
2,6-dimethyl-3-carboisopropoxy-4-(3-cyanophenyl)-5-carboxy-1,4-dihydropyridine;
2,6-dimethyl-3-carboisopropoxy-4-(3-trifluoromethylphenyl)-5-carboxy-1,4-dihydropyridine;
2,6-dimethyl-3-carboisopropoxy-4-(3-trifluoromethoxyphenyl)-5-carboxy-1,4-dihydropyridine;
2,6-dimethyl-3-methylthio-4-(2-nitrophenyl)-5-carboxy-1,4-dihydropyridine;
2,6-dimethyl-3-methylthio-4-(3-nitrophenyl)-5-carboxy-1,4-dihydropyridine;
2,6-dimethyl-3-methylthio-4-(3-chlorophenyl)-5-carboxy-1,4-dihydropyridine;
2,6-dimethyl-3-methylthio-4-(2-chlorophenyl)-5-carboxy-1,4-dihydropyridine;
2,6-dimethyl-3-methylthio-4-(2,3-dichlorophenyl)-5-carboxy-1,4-dihydropyridine;
2,6-dimethyl-3-methylthio-4-(4-methylphenyl)-5-carboxy-1,4-dihydropyridine;
2,6-dimethyl-3-methylthio-4-(3-cyanophenyl)-5-carboxy-1,4-dihydropyridine;
2,6-dimethyl-3-methylthio-4-(3-trifluoromethylphenyl)-5-carboxy-1,4-dihydropyridine;
2,6-dimethyl-3-methylthio-4-(3-trifluoromethoxyphenyl)-5-carboxy-1,4-dihydropyridine;
2,6-dimethyl-3-ethylthio-4-(2-nitrophenyl)-5-carboxy-1,4-dihydropyridine;
2,6-dimethyl-3-ethylthio-4-(3-nitrophenyl)-5-carboxy-1,4-dihydropyridine;

2,6-dimethyl-3-ethylthio-4-(3-chlorophenyl)-5-carboxy-1,4-dihydropyridine;
2,6-dimethyl-3-ethylthio-4-(2-chlorophenyl)-5-carboxy-1,4-dihydropyridine;
2,6-dimethyl-3-ethylthio-4-(2,3-dichlorophenyl)-5-carboxy-1,4-dihydropyridine;
2,6-dimethyl-3-ethylthio-4-(4-methylphenyl)-5-carboxy-1,4-dihydropyridine;
2,6-dimethyl-3-ethylthio-4-(3-cyanophenyl)-5-carboxy-1,4-dihydropyridine;
2,6-dimethyl-3-ethylthio-4-(3-trifluoromethylphenyl)-5-carboxy-1,4-dihydropyridine;
2,6-dimethyl-3-ethylthio-4-(3-trifluoromethylphenyl)-5-carboxy-1,4-dihydropyridine;
2,6-dimethyl-3-isopropylthio-4-(2-nitrophenyl)-5-carboxy-1,4-dihydropyridine;
2,6-dimethyl-3-isopropylthio-4-(3-nitrophenyl)-5-carboxy-1,4-dihydropyridine;
2,6-dimethyl-3-isopropylthio-4-(3-chlorophenyl)-5-carboxy-1,4-dihydropyridine;
2,6-dimethyl-3-isopropylthio-4-(2-chlorophenyl)-5-carboxy-1,4-dihydropyridine;
2,6-dimethyl-3-isopropylthio-4-(2,3-dichlorophenyl)-5-carboxy-1,4-dihydropyridine;
2,6-dimethyl-3-isopropylthio-4-(4-methylphenyl)-5-carboxy-1,4-dihydropyridine;
2,6-dimethyl-3-isopropylthio-4-(3-cyanophenyl)-5-carboxy-1,4-dihydropyridine;
2,6-dimethyl-3-isopropylthio-4-(3-trifluoromethylphenyl)-5-carboxy-1,4-dihydropyridine;
2,6-dimethyl-3-isopropylthio-4-(3-trifluoromethoxyphenyl)-5-carboxy-1,4-dihydropyridine;
2,6-dimethyl-3-methylsulfonyl-4-(2-nitrophenyl)-5-carboxy-1,4-dihydropyridine;
2,6-dimethyl-3-methylsulfonyl-4-(3-nitrophenyl)-5-carboxy-1,4-dihydropyridine;
2,6-dimethyl-3-methylsulfonyl-4-(3-chlorophenyl)-5-carboxy-1,4-dihydropyridine;
2,6-dimethyl-3-methylsulfonyl-4-(2-chlorophenyl)-5-carboxy-1,4-dihydropyridine;
2,6-dimethyl-3-methylsulfonyl-4-(2,3-dichlorophenyl)-5-carboxy-1,4-dihydropyridine;
2,6-dimethyl-3-methylsulfonyl-4-(4-methylphenyl)-5-carboxy-1,4-dihydropyridine;
2,6-dimethyl-3-methylsulfonyl-4-(3-cyanophenyl)-5-carboxy-1,4-dihydropyridine;
2,6-dimethyl-3-methylsulfonyl-4-(3-trifluoromethylphenyl)-5-carboxy-1,4-dihydropyridine;
2,6-dimethyl-3-methylsulfonyl-4-(3-trifluoromethoxyphenyl)-5-carboxy-1,4-dihydropyridine;
2,6-dimethyl-3-ethylsulfonyl-4-(2-nitrophenyl)-5-carboxy-1,4-dihydropyridine;
2,6-dimethyl-3-ethylsulfonyl-4-(3-nitrophenyl)-5-carboxy-1,4-dihydropyridine;
2,6-dimethyl-3-ethylsulfonyl-4-(3-chlorophenyl)-5-carboxy-1,4-dihydropyridine;
2,6-dimethyl-3-ethylsulfonyl-4-(2-chlorophenyl)-5-carboxy-1,4-dihydropyridine;
2,6-dimethyl-3-ethylsulfonyl-4-(2,3-dichlorophenyl)-5-carboxy-1,4-dihydropyridine;
2,6-dimethyl-3-ethylsulfonyl-4-(4-methylphenyl)-5-carboxy-1,4-dihydropyridine;
2,6-dimethyl-3-ethylsulfonyl-4-(3-cyanophenyl)-5-carboxy-1,4-dihydropyridine;
2,6-dimethyl-3-ethylsulfonyl-4-(3-trifluoromethylphenyl)-5-carboxy-1,4-dihydropyridine;
2,6-dimethyl-3-ethylsulfonyl-4-(3-trifluoromethoxyphenyl)-5-carboxy-1,4-dihydropyridine;
2,6-dimethyl-3-isopropylsulfonyl-4-(2-nitrophenyl)-5-carboxy-1,4-dihydropridine;
2,6-dimethyl-3-isopropylsulfonyl-4-(3-nitrophenyl)-5-carboxy-1,4-dihydropyridine;
2,6-dimethyl-3-isopropylsulfonyl-4-(3-chlorophenyl)-5-carboxy-1,4-dihydropyridine;
2,6-dimethyl-3-isopropylsulfonyl-4-(2-chlorophenyl)-5-carboxy-1,4-dihydropyridine;
2,6-dimethyl-3-isopropylsulfonyl-4-(2,3-dichlorophenyl)-5-carboxy-1,4-dihydropyridine;
2,6-dimethyl-3-isopropylsulfonyl-4-(4-methylphenyl)-5-carboxy-1,4-dihydropyridine;
2,6-dimethyl-3-isopropylsulfonyl-4-(3-cyanophenyl)-5-carboxy-1,4-dihydropyridine;
2,6-dimethyl-3-isopropylsulfonyl-4-(3-trifluoromethylphenyl)-5-carboxy-1,4-dihydropyridine;
2,6-dimethyl-3-isopropylsulfonyl-4-(3-trifluoromethoxyphenyl)-5-carboxy-1,4-dihydropyridine;
2,6-dimethyl-3-methylsulfinyl-4-(2-nitrophenyl)-5-carboxy-1,4-dihydropyridine;
2,6-dimethyl-3-methylsulfinyl-4-(3-nitrophenyl)-5-carboxy-1,4-dihydropyridine;
2,6-dimethyl-3-methylsulfinyl-4-(3-chlorophenyl)-5-carboxy-1,4-dihydropyridine;
2,6-dimethyl-3-methylsulfinyl-4-(2-chlorophenyl)-5-carboxy-1,4-dihydropyridine;
2,6-dimethyl-3-methylsulfinyl-4-(2,3-dichlorophenyl)-5-carboxy-1,4-dihydropyridine;
2,6-dimethyl-3-methylsulfinyl-4-(4-methylphenyl)-5-carboxy-1,4-dihydropyridine;
2,6-dimethyl-3-methylsulfinyl-4-(3-cyanophenyl)-5-carboxy-1,4-dihydropyridine;
2,6-dimethyl-3-methylsulfinyl-4-(3-trifluoromethylphenyl)-5-carboxy-1,4-dihydropyridine;
2,6-dimethyl-3-methylsulfinyl-4-(3-trifluoromethoxyphenyl)-5-carboxy-1,4-dihydropyridine;
2,6-dimethyl-3-ethylsulfinyl-4-(2-nitrophenyl)-5-carboxy-1,4-dihydropyridine;
2,6-dimethyl-3-ethylsulfinyl-4-(3-nitrophenyl)-5-carboxy-1,4-dihydropyridine;
2,6-dimethyl-3-ethylsulfinyl-4-(3-chlorophenyl)-5-carboxy-1,4-dihydropyridine;
2,6-dimethyl-3-ethylsulfinyl-4-(2-chlorophenyl)-5-carboxy-1,4-dihydropyridine;
2,6-dimethyl-3-ethylsulfinyl-4-(2,3-dichlorophenyl)-5-carboxy-1,4-dihydropyridine;
2,6-dimethyl-3-ethylsulfinyl-4-(4-methylphenyl)-5-carboxy-1,4-dihydropyridine;

2,6-dimethyl-3-ethylsulfinyl-4-(3-cyanophenyl)-5-carboxy-1,4-dihydropridine;
2,6-dimethyl-3-ethylsulfinyl-4-(3-trifluoromethylphenyl)-5-carboxy-1,4-dihydropyridine;
2,6-dimethyl-3-ethylsulfinyl-4-(3-trifluoromethoxyphenyl)-5-carboxy-1,4-dihydropyridine;
2,6-dimethyl-3-isopropylsulfinyl-4-(2-nitrophenyl)-5-carboxy-1,4-dihydropyridine;
2,6-dimethyl-3-isopropylsulfinyl-4-(3-nitrophenyl)-5-carboxy-1,4-dihydropyridine;
2,6-dimethyl-3-isopropylsulfinyl-4-(3-chlorophenyl)-5-carboxy-1,4-dihydropyridine;
2,6-dimethyl-3-isopropylsulfinyl-4-(2-chlorophenyl)-5-carboxy-1,4-dihydropyridine;
2,6-dimethyl-3-isopropylsulfinyl-4-(2,3-dichlorophenyl)-5-carboxy-1,4-dihydropyridine;
2,6-dimethyl-3-isopropylsulfinyl-4-(4-methylphenyl)-5-carboxy-1,4-dihydropyridine;
2,6-dimethyl-3-isopropylsulfinyl-4-(3-cyanophenyl)-5-carboxy-1,4-dihydropyridine;
2,6-dimethyl-3-isopropylsulfinyl-4-(3-trifluoromethylphenyl)-5-carboxy-1,4-dihydropyridine;
2,6-dimethyl-3-isopropylsulfinyl-4-(3-trifluoromethoxyphenyl)-5-carboxy-1,4-dihydropyridine;
2,6-dimethyl-3-methoxyethoxycarbonyl-4-(2-nitrophenyl)-5-carboxy-1,4-dihydropyridine;
2,6-dimethyl-3-methoxyethoxycarbonyl-4-(3-chlorophenyl)-5-carboxy-1,4-dihydropyridine;
2,6-dimethyl-3-methoxyethoxycarbonyl-4-(2-chlorophenyl)-5-carboxy-1,4-dihydropyridine;
2,6-dimethyl-3-methoxyethoxycarbonyl-4-(2,3-dichlorophenyl)-5-carboxy-1,4-dihydropyridine;
2,6-dimethyl-3-methoxyethoxycarbonyl-4-(4-methylphenyl)-5-carboxy-1,4-dihydropyridine;
2,6-dimethyl-3-methoxyethoxycarbonyl-4-(3-cyanophenyl)-5-carboxy-1,4-dihydropyridine;
2,6-dimethyl-3-methoxyethoxycarbonyl-4-(3-trifluoromethylphenyl)-5-carboxy-1,4-dihydropyridine;
2,6-dimethyl-3-methoxyethoxycarbonyl-4-(3-trifluoromethoxyphenyl)-5-carboxy-1,4-dihydropyridine;
2,6-dimethyl-3-methoxyethoxycarbonyl-4-(3-methoxyphenyl)-5-carboxyl-1,4-dihydropyridine;
2,6-dimethyl-3-methoxyethoxycarbonyl-4-(2-thienyl)-5-carboxy-1,4-dihydropyridine;
2,6-dimethyl-3-methoxyethoxycarbonyl-4-(5-methylthien-2-yl)-5-carboxy-1,4-dihydropyridine;
2,6-dimethyl-3-cyano-4-(2-nitrophenyl)-5-carboxy-1,4-dihydropyridine;
2,6-dimethyl-3-cyano-4-(3-nitrophenyl)-5-carboxy-1,4-dihydropyridine;
2,6-dimethyl-3-cyano-4-(3-chlorophenyl)-5-carboxy-1,4-dihydropyridine;
2,6-dimethyl-3-cyano-4-(2-chlorophenyl)-5-carboxy-1,4-dihydropyridine;
2,6-dimethyl-3-cyano-4-(2,3-dichlorophenyl)-5-carboxy-1,4-dihydropyridine;
2,6-dimethyl-3-cyano-4-(4-methylphenyl)-5-carboxy-1,4-dihydropyridine;
2,6-dimethyl-3-cyano-4-(3-cyanophenyl)-5-carboxy-1,4-dihydropyridine;
2,6-dimethyl-3-cyano-4-(3-trifluoromethylphenyl)-5-carboxy-1,4-dihydropyridine;
2,6-dimethyl-3-cyano-4-(3-trifluoromethoxyphenyl)-5-carboxy-1,4-dihydropyridine;
2,6-dimethyl-3-cyano-4-(3-methoxyphenyl)-5-carboxy-1,4-dihydropyridine;
2,6-dimethyl-3-cyano-4-(2-thienyl)-5-carboxy-1,4-dihydropyridine; and
2,6-dimethyl-3-cyano-4-(5-methylthien-2-yl)-5-carboxy-1,4-dihydropyridine.

(C) The mother liquor obtained in part (A) above was then evaporated, and the residue suspended in $H_2O$ (400 mL) containing NaOH (22 mL of 35% solution). The aqueous phase was washed with $CH_2Cl_2$ (2 x 200 mL) and acidified with HCl (12N, 22 mL). The precipitate was filtered, washed with water, and dried to obtain nearly pure (-) 2,6-dimethyl-3-carbomethyoxy-4-(3-nitrophenyl)-5-carboxy-1,4-dihydropyridine, $[\alpha]^{20}_{D}$ = -17° (c = 0.5, acetone). This acid (10 g) was mixed with cinchonine (8.9 g) dissolved in hot EtOH (100 mL). When both compounds were nearly solubilized, the mixture was allowed to stand overnight. The resulting precipitate was filtered, acidified, washed and dried to yield pure (-) 2,6-dimethyl-3-carbomethoxy-4-(3-nitrophenyl)-5-carboxy-1,4-dihydropyridine, $[\alpha]^{20}_{D}$ = -20.4° (c = 0.5, acetone).

(D) Similarly, proceeding as in part (C) above but substituting the mother liquors obtained in part (B) above for the mother liquor obtained from part (A), the corresponding compounds are prepared.

## EXAMPLE 3

(Preparation of Active Compounds)

(A) (1) A solution of (+)-2,6-dimethyl-3-methoxycarbonyl-4-(3-nitrophenyl)-5-carboxy-1,4-dihydropyridine ($[\alpha]_D$ = +27.5°, 8.0 g) in $CH_2Cl_2$ at 0°C was treated with $PCl_5$ (5.3 g). To this mixture was added a solution of (R)-4-(2,2-dimethyl-1,3-dioxolan4-yl)methoxyphenylethan-2-ol (6.4 g, $[\alpha]_D$ = +9.11° in $CHCl_3$) and triethylamine (3.8 mL) in $CH_2Cl_2$ to yield 2,6-dimethyl-3-methoxycarbonyl-4-(3-nitrophenyl)-5-(2-[4-(2,2-dimethyl-1,3-dioxolan-4-yl)methoxyphenyl)ethoxycarbonyl)-1,4-dihydropyridine.

(2) A solution of 21 g of 2,6-dimethyl-3-methoxycarbonyl-4-(3-nitrophenyl)-5-(2-[4-(2,2-dimethyl-1,3-dioxolan-4-yl)methoxyphenyl]ethoxycarbonyl)-1,4-dihydropyridine in 150 mL of acetone and 50 mL of water

is treated with 10 mL of hydrochloric acid and the mixture heated at reflux for 6 h. Water (500 mL) is added and the mixture was extracted with ether. The ether layer is dried over $Na_2SO_4$ and evaporated to an oil which is purified by medium pressure chromatography on silica gel (90% ethyl acetate-hexane) to give 2,6-dimethyl-3-methoxycarbonyl-4-(3-nitrophenyl)-5-(2-[4-(2,3-dihydroxypropoxy)phenyl]ethoxycarbonyl)-1,4-dihydropyridine, m.p. 117-118°C, $[\alpha]_D$ = -33.5°.

(B) Using the procedure of part (A) one isomer of 2,6-dimethyl-3-methoxycarbonyl-4-(3-nitrophenyl)-5-carboxy-1,4-dihydropyridine is reacted with β-(N-benzyl-N-methylamino)ethanol in the presence of thionyl chloride to provide the isomer of 2,6-dimethyl-3-methoxycarbonyl-4-(3-nitrophenyl)-5-[2-(N-methyl-N-benzylamino)-ethoxycarbonyl]-1,4-dihydropyridine, mp = 135-138°C.

(C) 4-(2-tetrahydropyran-2-yloxyethyl)phenol (72.1 g, 0.32 mol) was added to a solution of the appropriate isomer of 2,6-dimethyl-3-carbomethoxy-4-(3-nitrophenyl)-5-(3-bromopropoxycarbonyl)-1,4-dihydropyridine (105 g, 0.23 mol) and $K_2CO_3$ (48 g, 0.35 mol) in acetone (700 ml), and the mixture heated to reflux overnight. After cooling, the solid was filtered, the solvent removed under reduced pressure, and the residue solubilized in $CH_2Cl_2$ (1 L). The organic layer was washed with 10% NaOH (2 x 100 mL) and water (2 x 100 ml) and dried over sodium sulfate. The solvent was evaporated under reduced pressure to provide an oil which was purified by flash chromatography (EtOAc:$CH_2Cl_2$ 15:85) to yield the appropriate isomer of 2,6-dimethyl-3-carbomethoxy-4-(3-nitrophenyl)-5-(3-[4-(2-tetrahydropyran-2-y(oxyethyl)-phenoxy]-propoxycarbonyl)-1,4-dihydropyridine (mp <50°C).

(D) Similarly, proceeding as in part (A)(1) above but substituting ethanol or isopropanol for 4-(2,2-dimethyl-1,3-dioxolan-4-yl)methoxyphenylethan-2-ol, the following optically active compounds are prepared:
2,6-dimethyl-3-methoxycarbonyl-4-(3-nitrophenyl)-5-ethoxycarbonyl-1,4-dihydropyridine;
2,6-dimethyl-3-methoxycarbonyl-4-(3-nitrophenyl)-5-isopropoxycarbonyl-1,4-dihydropyridine; and

(E) Similarly, proceeding as in parts (A)(1) and (D) above but substituting (+)-2,6-dimethyl-3-methoxysulfonyl-4-(3-nitrophenyl)-5-carboxy-1,4-dihydropyridine, (-)-2,6-dimethyl-3-methoxysulfonyl-4-(3-nitrophenyl)-5-carboxy-1,4-dihydropyridine, (+)-2,6-dimethyl-3-dimethylphosphonyl-4-(3-nitrophenyl)-5-carboxy-1,4-dihydropyridine, (-)-2,6-dimethyl-3-dimethylphosphonyl-4-(3-nitrophenyl)-5-carboxy-1,4-dihydropyridine, (+)-2,6-dimethyl-3-diethylphosphonyl-4-(3-nitrophenyl)-5-carboxy-1,4-dihydropyridine, (-)-2,6-dimethyl-3-diisopropylphosphonyl-4-(3-nitrophenyl)-5-carboxy-1,4-dihydropyridine, (+)-2,6-dimethyl-3-nitro-4-(2-trifluoromethylphenyl)-5-carboxy-1,4-dihydropyridine, (-)-2,6-dimethyl-3-nitro-4-(2-trifluoromethylphenyl)-5-carboxy-1,4-dihydropyridine, (+)-2,6-dimethyl-3-nitro-4-(4-benzodioxazolyl)-5-carboxy-1,4-dihydropyridine, (-)-2,6-dimethyl-3-nitro-4-(4-benzodioxazolyl)-5-carboxy-1,4-dihydropyridine, (+)-2,6-dimethyl-3-cyano-4-(2-trifluoromethylphenyl)-5-carboxy-1,4-dihydropyridine, or (-)-2,6-dimethyl-3-cyano-4-(2-trifluoromethylphenyl)-5-carboxy-1,4-dihydropyridine, for (+)-2,6-dimethyl-3-methoxycarbonyl-4-(3-nitrophenyl)-5-carboxy-1,4-dihydropyridine, and using methanol, ethanol, isopropanol, the following compounds are prepared:
(+)-2,6-dimethyl-3-methoxysulfonyl-4-(3-nitrophenyl)-5-carbomethoxy-1,4-dihydropyridine;
(-)-2,6-dimethyl-3-methoxysulfonyl-4-(3-nitrophenyl)-5-carbomethoxy-1,4-dihydropyridine;
(+)-2,6-diemthyl-3-nitro-4-(2-trifluoromethylphenyl)-5-carbomethoxy-1,4-dihydropyridine;
(-)-2,6-dimethyl-3-nitro-4-(2-trifluoromethylphenyl)-5-carbomethoxy-1,4-dihydropyridine;
(+)-2,6-dimethyl-3-nitro-4-(4-benzodioxazolyl)-5-carbomethoxy-1,4-dihydropyridine;
(-)-2,6-dimethyl-3-nitro-4-(4-benzodioxazolyl)-5-carbomethoxy-1,4-dihydropyridine;
(+)-2,6-dimethyl-3-cyano-4-(2-trifluoromethylphenyl)-5-carbomethoxy-1,4-dihydropyridine;
(-)-2,6-dimethyl-3-cyano-4-(2-trifluoromethylphenyl)-5-carbomethoxy-1,4-dihydropyridine;
(+)-2,6-dimethyl-3-methoxysulfonyl-4-(3-nitrophenyl)-5-carboethoxy-1,4-dihydropyridine;
(-)-2,6-dimethyl-3-methoxysulfonyl-4-(3-nitrophenyl)-5-carboethoxy-1,4-dihydropyridine;
(+)-2,6-dimethyl-3-nitro-4-(2-trifluoromethylphenyl)-5-carboethoxy-1,4-dihydropyridine;
(-)-2,6-dimethyl-3-nitro-4-(2-trifluoromethylphenyl)-5-carboethoxy-1,4-dihydropyridine;
(+)-2,6-dimethyl-3-nitro-4-(4-benzodioxazolyl)-5-carboethoxy-1,4-dihydropyridine;
(-)-2,6-dimethyl-3-nitro-4-(4-benzodioxazolyl)-5-carboethoxy-1,4-dihydropyridine;
(+)-2,6-dimethyl-3-cyano-4-(2-trifluoromethylphenyl)-5-carboethoxy-1,4-dihydropyridine;
(1)-2,6-dimethyl-3-cyano-4-(2-trifluoromethylphenyl)-5-carboethoxy-1,4-dihydropyridine;
(+)-2,6-dimethyl-3-methoxysulfonyl-4-(3-nitrophenyl)-5-carboisopropoxy-1,4-dihydropyridine;
(-)-2,6-dimethyl-3-methoxysulfonyl-4-(3-nitrophenyl)-5-carboisopropoxy-1,4-dihydropyridine;
(+)-2,6-dimethyl-3-nitro-4-(2-trifluoromethylphenyl)-5-carboisopropoxy-1,4-dihydropyridine;
(-)-2,6-dimethyl-3-nitro-4-(2-trifluoromethylphenyl)-5-carboisopropoxy-1-4-dihydropyridine;
(+)-2,6-dimethyl-3-nitro-4-(4-benzodioxazolyl)-5-carboisopropoxy-1,4-dihydropyridine;
(-)-2,6-dimethyl-3-nitro-4-(4-benzodioxazolyl)-5-carboisopropoxy-1,4-dihydropyridine;
(+)-2,6-dimethyl-3-cyano-4-(2-trifluoromethylphenyl)-5-carboisopropoxy-1,4-dihydropyridine;

(-)-2,6-dimethyl-3-cyano-4-(2-trifluoromethylphenyl)-5-carboisopropoxy-1,4-dihydropyridine;
( + )-2,6-dimethyl-3-dimethylphosphonyl-4-(3-nitrophenyl)-5-carbomethoxy-1,4-dihydropyridine;
(-)-2,6-dimethyl-3-dimethylphosphonyl-4-(3-nitrophenyl)-5-carbomethoxy-1,4-dihydropyridine;
( + )-2,6-dimethyl-3-diethylphosphonyl-4-(3-nitrophenyl)-5-carbomethoxy-1,4-dihydropyridine; and
(-)-2,6-dimethyl-3-diisopropylphosphonyl-4-(3-nitrophenyl)-5-carbomethoxy-1,4-dihydropyridine.

## Claims

1. A process for preparing a 1,4-dihydropyridine derivative having a carboxylic acid or carboxylic acid function in position 5 of the 1,4-dihydropyridine ring and an aryl, heterocyclyl, fused heterocyclyl, optionally substituted with one, two or three substituents, in position 4 of the dihydropyridine ring, or a salt thereof, which process comprises forming a salt mixture of a compound of formula (X)

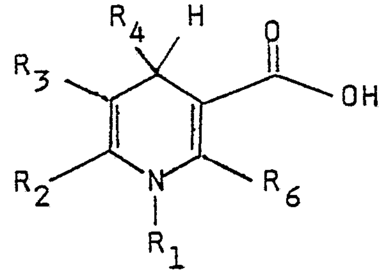

(X)

or a salt thereof
wherein
$R_1$ is H or lower alkyl;
$R_2$ and $R_6$ are each independently lower alkyl, aryl, or arylalkyl;
$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl; and
$R_4$ is aryl, hetercyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$;
with a resolving agent in a solvent selected from dimethyl formamide, ethanol, and acetonitrile, optionally in admixture with water, at a temperature of from 50°C
to the reflux temperature of said solvent, optionally adding water during this elevated temperature, such that the ratio of 1,4-dihydropyridine derivative to resolving agent is about 1:2 to about 2:1 and the mixture contains about 10% to about 50% water, wherein said salt mixture comprises

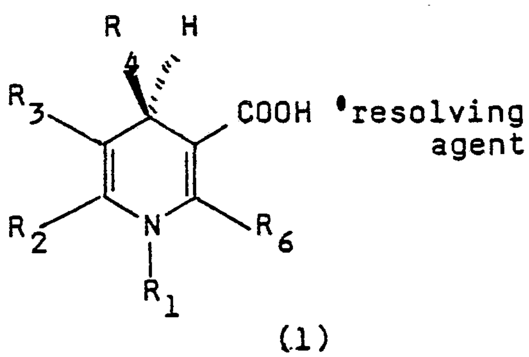

(1)

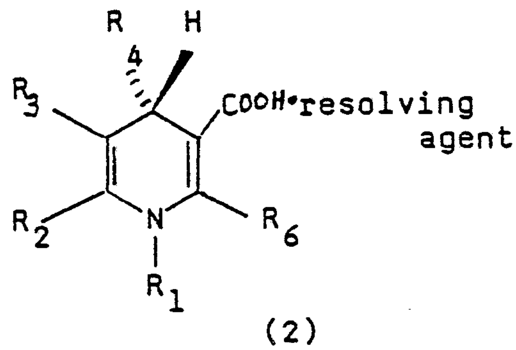

(2)

or a salt thereof
wherein $R_1$, $R_2$, $R_3$, $R_4$, and $R_6$ are as defined above.

2. The process of claim 1 wherein said 1,4-dihydropyridine derivative is a compound of the formula (1A) or (2A)

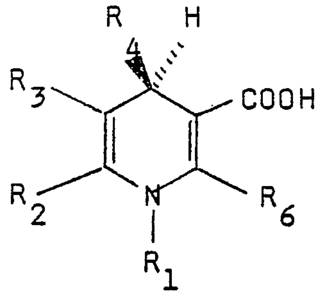

(1A)

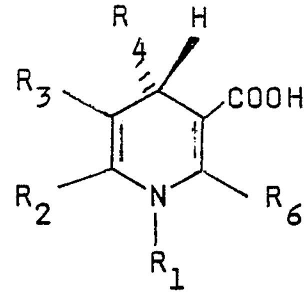

(2A)

or a salt thereof
wherein
$R_1$ is H or lower alkyl;
$R_2$ and $R_6$ are each independently lower alkyl, aryl, or arylalkyl;
$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl; and
$R_4$ is aryl, heterocyclyl, or fused-ring heterocyclyl, optionally substituted with one, two, or three halo, $NO_2$, CN, lower alkyl, lower alkoxy, lower alkylamino, $CF_3$, $OCH_2F$, or $OCF_3$.

3. The process of Claim 1 or 2 wherein the resolving agent comprises cinchonidine, cinchonine, quinine, quinidine, strychnine, brucine, dehydroabietylamine, cinchonicine, L-2-amino-1-propanol, D-amphetamine, glucosamine, conessine, anabasine, D-ephedrine, or an N-alkyl-D-glucamine, wherein alkyl is C1 to C10.

4. The process of any one of Claims 1 to 3 wherein the water is added during the elevated temperatures portion of the reaction.

5. The process of any one of Claims 1 to 4 which further comprises

a) separating the compound represented by the formula (1) from the compound represented by the formula (2), and

b) optionally purifying the isolated compound of formula (1) or (2).

6. The process of claim 5 which further comprises cleaving a compound of formula (1) or (2) to form a compound of the formula (1A) or (2A).

7. The process of Claim 6 wherein said cleavage is performed with strong base followed by strong acid, or with strong acid.

8. The process of any one of Claims 1 to 7 which further comprises the steps of recovering a non-racemic mixture of (+)-or (-)-1,4-dihydropyridine derivative from the mother liquors, racemizing the mixture, and recycling said racemized mixture.

9. The process of any one of Claims 5 to 7 which further comprises the step of racemizing the undesired isomer and recycling said racemized mixture.

10. A compound of the formula (1) or (2)

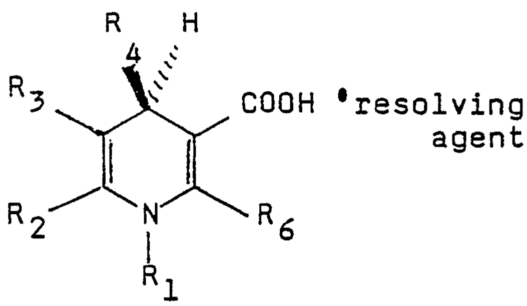

(1)

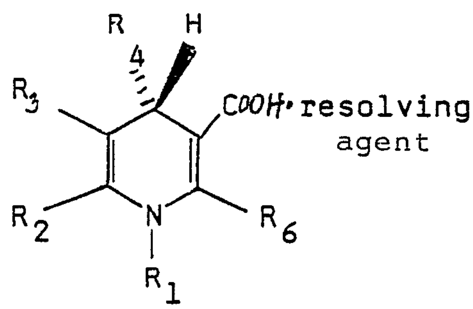

(2)

or a salt thereof
wherein
$R_1$ is H or lower alkyl;
$R_2$ and $R_6$ are each independently lower alkyl, aryl, or arylalkyl;
$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl;

$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$; and

resolving agent comprises an optically active amine base such as cinchonidine, cinchonine, quinine, quinidine, strychnine, brucine, morphine, D-α-phenylethylamine, L-arginine, dehydroabietylamine, cinchonicine, L-2-amino-1-propanol, D-amphetamine, glucosamine, conessine, anabasine, D-ephedrine, or an N-alkyl-D-glucamine wherein alkyl is C1 to C10.

11. A compound of Claim 10 wherein $R_1$ is H and $R_2$ and $R_6$ are each methyl.

12. A compound of Claim 11 wherein $R_3$ is $CO_2R_5$ and $R_5$ is methyl, ethyl, isopropyl, or methoxyethyl.

13. A compound of Claim 12 wherein $R_4$ is phenyl optionally substituted with one, two, or three substituents independently selected from halo, $NO_2$, CN, lower alkyl, lower alkoxy, loweralkylamino, $CF_3$, $OCH_2F$, and $OCF_3$.

14. The process of any of claims 1 to 9 wherein the 1,4-dihydropyridine derivatives are represented by the formula (3)

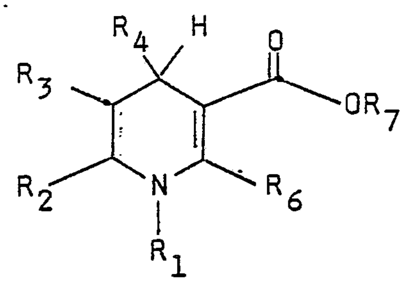

(3)

or a salt thereof

wherein

$R_1$ is H or lower alkyl;

$R_2$ and $R_6$ are each independently lower alkyl, aryl, or arylalkyl;

$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl;

$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$;and

$R_7$ is an alcohol derivative of the general formula $HOR_8$ wherein $R_8$ is $-O(CH_2)_nR_9$ wherein n is 2 or 3 and $R_9$ is NR'R" wherein R' is lower alkyl and R" is arylalkyl, phenyl substituted in the para position with a 2,3-dihydroxypropoxy group, or phenoxy substituted in the para position with a 2-(tetrahydropyran-2-yloxy)ethyl group,

which comprises the following additional steps

a) reacting a compound of formula (1A) or (2A) with the appropriate alcohol derivative to form a compound of formula (3); and

b) optionally converting a compound of formula (3) to a salt.

15. The process of Claim 14 wherein the compound of formula (3) is selected from: 2,6-dimethyl-3-methoxycarbonyl-4-(3-nitrophenyl)-5-[2-(N-methyl-N-benzylamino)ethoxy-carbonyl]-1,4-dihydropyridine, 2,6-dimethyl-3-methoxycarbonyl-4-(3-nitrophenyl)-5-(2-[4-(2,3-dihydroxypropoxy)phenyl]ethoxycarbonyl)-1,4-dihydropyridine; and 2,6-dimethyl-3-methoxycarbonyl-4-(3-nitrophenyl)-5-(3-[4-(2-tetrahydropyran-2-yloxy-ethyl)phenoxy]propoxycarbonyl)-1,4-dihydropyridine.

16. The use of the compounds of Claim 10 or the compojnds prepared by the processes of claims 2 to 9 in the preparation of a 1,4-dihydropyridine derivatives of the formula (3)

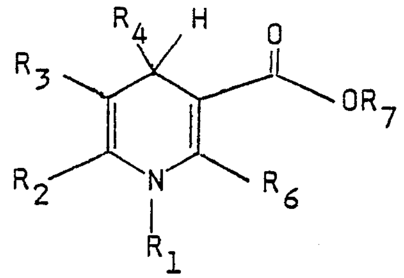

(3)

or a salt thereof
wherein
$R_1$ is H or lower alkyl;
$R_2$ and $R_6$ are each independently lower alkyl, aryl, or arylalkyl;
$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl;
$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$;and
$R_7$ is an alcohol derivative of the general formula $HOR_8$ wherein $R_8$ is $-O(CH_2)_nR_9$ wherein n is 2 or 3 and $R_9$ is NR'R" wherein R' is lower alkyl and R" is arylalkyl, phenyl substituted in the para position with a 2,3-dihydroxypropoxy group, or phenoxy substituted in the para position with a 2-(tetrahydropyran-2-yloxy)ethyl group.

17. 1,4-Dihydropyridine derivatives of the formula (3)

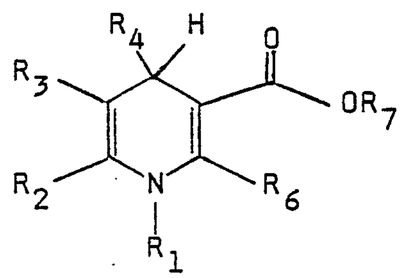

(3)

or a salt thereof
wherein
$R_1$ is H or lower alkyl;
$R_2$ and $R_6$ are each independently lower alkyl, aryl, or arylalkyl;
$R_3$ is CN, $NO_2$, $CO_2R_5$, $CONHR_5$, $SO_2R_5$, or $P(O)OR_5)_2$, where $R_5$ is lower alkyl, lower alkoxyalkyl, aryl, or arylalkyl;
$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$;and
$R_7$ is an alcohol derivative of the general formula $HOR_8$ wherein $R_8$ is $-O(CH_2)_nR_9$ wherein n is 2 or 3 and $R_9$ is NR'R" wherein R' is lower alkyl and R" is arylalkyl, phenyl substituted in the para position with a 2,3-dihydroxypropoxy group, or phenoxy substituted in hte para position with a 2-(tetrahydropyran-2-yloxy)ethyl group
prepared from a compound of formula (1) or (2)

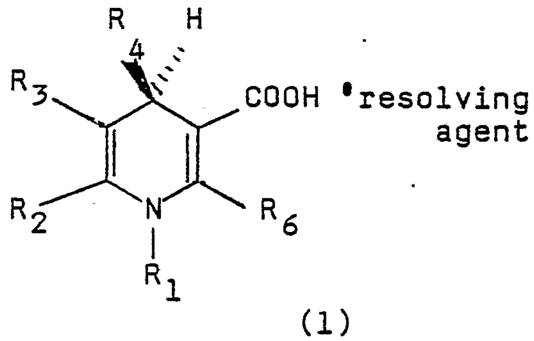

(1)

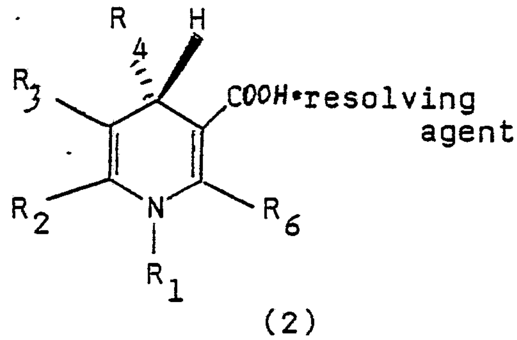

(2)

or a salt thereof
wherein
$R_1$ is H or lower alkyl;
$R_2$ and $R_6$ are each independently lower alkyl, aryl, or arylalkyl;
$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl;
$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$; and
said resolving agent comprises an optically active amine base such as cinchonidine, cinchonine, quinine, quinidine, strychnine, brucine, morphine, D-α-phenylethylamine, L-arginine, dehydroabietylamine, cinchonicine, L-2-amino-1-propanol, D-amphetamine, glucosamine, conessine, anabasine, D-ephedrine, or an N-alkyl-D-glucamine wherein alkyl is C1 to C10.

18. 1,4-Dihydropyridine derivatives of the formula (3)

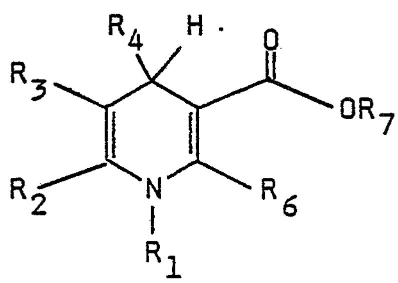

(3)

or a salt thereof
wherein
$R_1$ is H or lower alkyl;
$R_2$ and $R_6$ are each independently lower alkyl, aryl, or arylalkyl;
$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl;
$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$;and
$R_7$ is an alcohol derivative of the general formula $HOR_8$ wherein $R_8$ is $-O(CH_2)_nR_9$ wherein n is 2 or 3 and $R_9$ is NR′R″ wherein R′ is lower alkyl and R″ is arylalkyl, phenyl substituted in the para position with a 2,3-dihydroxypropyoxy group, or phenoxy substituted in the para position with a 2-(tetrahydropyran-2-yloxy)-ethyl group
containing detectable amounts of a resolving agent.

19. A compound of Claim 18 wherein said resolving agent comprises an optically active amine base such as cinchonidine, cinchonine, quinine, quinidine, strychnine, brucine, morphine, D-α-phenylethylamine, L-arginine, dehydroabietylamine, cinchonicine, L-2-amino-1-propanol, D-amphetamine, glucosamine, conessine, anabasine, D-ephedrine, or an N-alkyl-D-glucamine wherein alkyl is C1 to C10.

20. 1,4-Dihydropyridine derivatives of the formula (3)

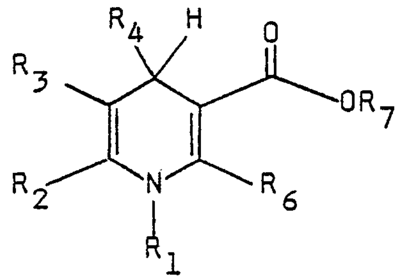

(3)

or a salt thereof

wherein

$R_1$ is H or lower alkyl;

$R_2$ and $R_6$ are each independently lower alkyl, aryl, or arylalkyl;

$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl;

$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$;and

$R_7$ is an alcohol derivative of the general formula $HOR_8$ wherein $R_8$ is $-O(CH_2)_nR_9$ wherein n is 2 or 3 and $R_9$ is NR'R" wherein R' is lower alkyl and R" is arylalkyl, phenyl substituted in the para position with a 2,3-dihydroxypropoxy group, or phenoxy substituted in the para position with a 2-(tetrahydropyran-2-yloxy)ethyl group

containing detectable amounts of a compound of formula (1) or (2)

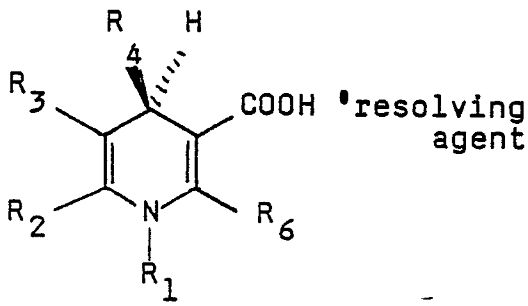

(1)

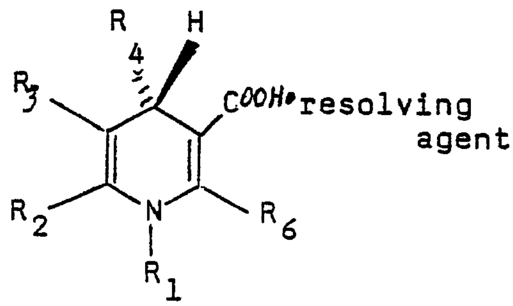

(2)

or a salt thereof

wherein

$R_1$ is H or lower alkyl;

$R_2$ and $R_6$ are each independently lower alkyl, aryl, or arylalkyl;

$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl;

$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$; and

resolving agent comprises an optically active amine base such as cinchonidine, cinchonine, quinine, quinidine, strychnine, brucine, morphine, D-α-phenylethylamine, L-arginine, dehydroabietylamine, cinchonicine, L-2-amino-1-propanol, D-amphetamine, glucosamine, conessine, anabasine, D-ephedrine, or an N-alkyl-D-glucamine wherein alkyl is C1 to C10.

21. A compound of formula (1) or (2)

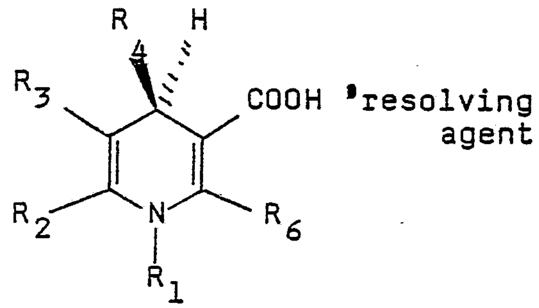

(1)

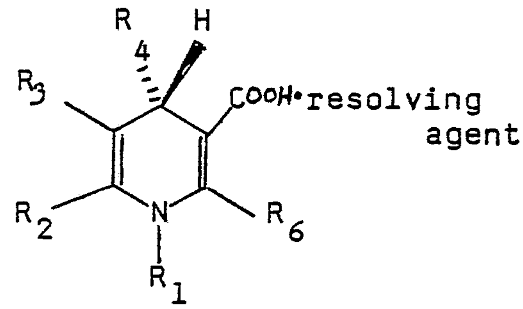

(2)

or a salt thereof
wherein
$R_1$ is H or lower alkyl;
$R_2$ and $R_6$ are each independently lower alkyl, aryl, or arylalkyl;
$R_3$ is CN, $No_2$, $CO_2R_5$, $CONHR_5$, $SO_2R_5$, or $P(O)(OR_5)_2$, where $R_5$ is lower alkyl, lower alkoxyalkyl, aryl, or arylalkyl;
$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$; and
resolving agent comprises an optionally active amine base such as cinchonidine, cinchonine, quinine, quinidine, strychnine, brucine, morphine, D-α-phenylethylamine, L-arginine, dehydroabietylamine, cinchonicine, L-2-amino-1-propanol, D-amphetamine, glucosamine, conessine, anabasine, D-ephedrine, or an N-alkyl-D-glucamine wherein alkyl is C1 to C10 in admixture with a 1,4-dihydropyridine derivative of the formula (3)

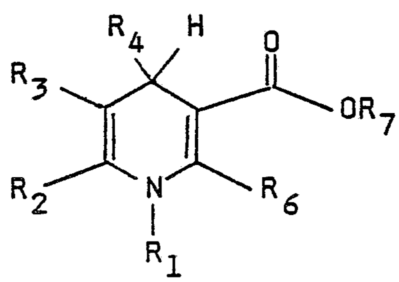

(3)

or a salt thereof
wherein
$R_1$ is H or lower alkyl;
$R_2$ and $R_6$ are each independently lower alkyl, aryl, or arylalkyl;
$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl;
$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$;and
$R_7$ is an alcohol derivative of the general formula $HOR_8$ wherein $R_8$ is $-O(CH_2)_nR_9$ wherein n is 2 or 3 and $R_9$ is NR′R″ wherein R′ is lower alkyl and R″ is arylalkyl, phenyl substituted in the para position with a 2,3-dihydroxypropoxy group, or phenoxy substituted in the para position with a 2-(tetrahydropyran-2-yloxy)ethyl group.

22. 1,4-Dihydropyridine derivative of any one of Claims 18 to 21 wherein the compound of formula (3) is selected from:
2,6-dimethyl-3-methoxycarbonyl-4-(3-nitrophenyl)-5-[2-(N-methyl-N-benzylamino)ethoxy-carbonyl]-1,4-dihydropyridine; 2,6-dimethyl-3-methoxycarbonyl-4-(3-nitrophenyl)-5-(2-[4-(2,3-dihydroxypropoxy)phenyl]-ethoxy-carbonyl)-1,4-dihydropyridine;and
2,6-dimethyl-3-methoxycarbonyl-4-(3-nitrophenyl)-5-(3-[4-(2-tetrahydropyran-2-yloxyethyl)phenoxy]-propoxycarbonyl)-1,4-dihydropyridine.

23. A mixture of the salts of a 1,4-dihydropyridine derivative of formula (X) with a resolving agent.

<u>Claims for the following contracting state: ES</u>

1. A process for preparing a 1,4-dihydropyridine derivative having a carboxylic acid or carboxylic acid function in position 5 of the 1,4-dihydropyridine ring and an aryl, heterocyclyl, fused heterocyclyl, optionally substituted with one, two or three substituents, in position 4 of the dihydropyridine ring, or a salt thereof, which process comprises forming a salt mixture of a compound of formula (X)

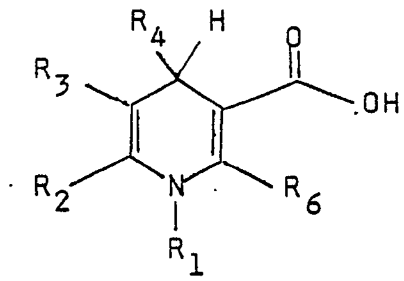

(X)

or a salt thereof
wherein
$R_1$ is H or lower alkyl;
$R_2$ and $R_6$ are each independently lower alkyl, aryl, or arylalkyl;
$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl; and
$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$;
with a resolving agent in a solvent selected from dimethyl formamide, ethanol, and acetonitrile, optionally in admixture with water, at a temperature of from 50°C to the reflux temperature of said solvent, optionally adding water during this elevated temperatures, such that the ratio of 1,4-dihydropyridine derivative to resolving agent is about 1:2 to about 2:1 and the mixture contains about 10% to about 50% water, wherein said salt mixture comprises

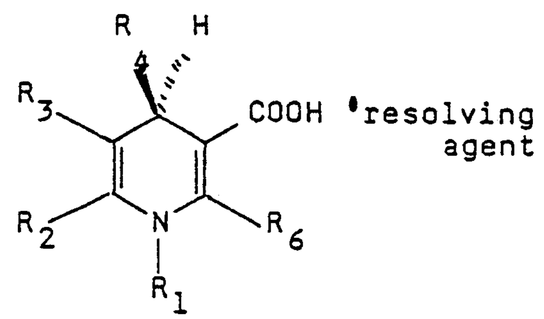

(1)

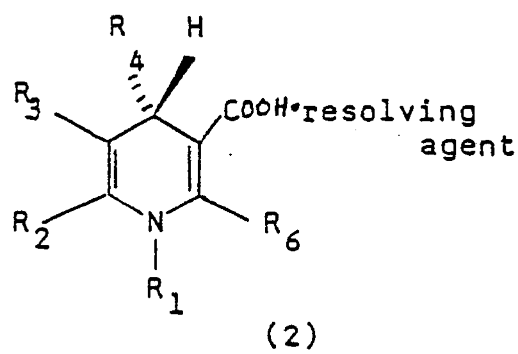

(2)

or a salt thereof
wherein $R_1$, $R_2$, $R_3$, $R_4$, and $R_6$ are as defined above.

2. The process of claim 1 wherein said 1,4-dihydropyridine derivative is a compound of the formula (1A) or (2A)

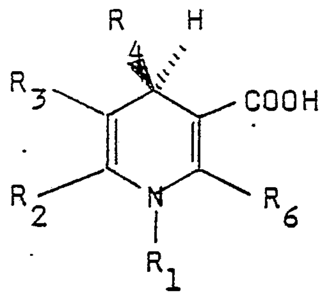

(1A)

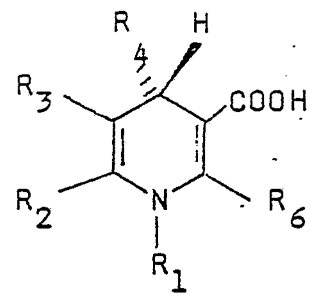

(2A)

or a salt thereof

wherein

$R_1$ is H or lower alkyl;

$R_2$ and $R_6$ are each independently lower alkyl, aryl, or arylalkyl;

$R_3$ is CN, $NO_2$, $CO_2R_5$, $CONHR_5$, $So_2R_5$, or $P(O)(OR_5)_2$, where $R_5$ is lower alkyl, lower alkoxyalkyl, aryl, or arylalkyl; and

$R_4$ is aryl, heterocyclyl, or fused-ring heterocyclyl, optionally substituted with one, two, or three halo, $NO_2$, CN, lower alkyl, lower alkoxy, lower alkylamino, $CF_3$, $OCH_2F$, or $OCF_3$.

3. The process of Claim 1 or 2 wherein the resolving agent comprises cinchonidine, cinchonine, quinine, quinidine, strychnine, brucine, dehydroabietylamine, cinchonicine, L-2-amino-1-propanol, D-amphetamine, glucosamine, conessine, anabasine, D-ephedrine, or an N-alkyl-D-glucamine, wherein alkyl is C1 to C10.

4. The process of any one of Claims 1 to 3 wherein the water is added during the elevated tempature portion of the reaction.

5. The process of any one of Claims 1 to 4 which further comprises

a) separating the compound represented by the formula (1) from the compound represented by the formula (2), and

b) optionally purifying the isolated compound of formula (1) or (2).

6. The process of claim 5 which further comprises cleaving a compound of formula (1) or (2) to form a compound of the formula (1a) or (2a).

7. The process of Claim 6 wherein said cleavage is performed with strong base followed by strong acid, or with strong acid.

8. The process of any one of Claims 1 to 7 which further comprises the steps of recovering a non-racemic mixture of (+)-or (-)-1,4-dihydropyridine derivative from the mother liquors, racemizing the mixture, and recycling said racemized mixture.

9. The process of any one of Claims 5 to 7 which further comprises the step of racemizing the undesired isomer and recycling said racemized mixture.

10. The process of any one of Claims 1 to 9 wherein $R_1$ is H and $R_2$ and $R_6$ are each methyl.

11. The process of Claim 10 wherein $R_3$ is $CO_2R_5$ and $R_5$ is methyl, ethyl, isopropyl, or methoxyethyl.

12. The process of Claim 11 wherein $R_4$ is phenyl optionally substituted with one, two, or three substituents independently selected from halo, $NO_2$, CN, lower alkyl, lowre alkoxy, loweralkylamino, $CF_3$, $OCH_2F$, and $OCF_3$.

13. The process of any one of Claims 1 to 12 wherein the 1,4-dihydropyridine derivatives are represented by the formula (3)

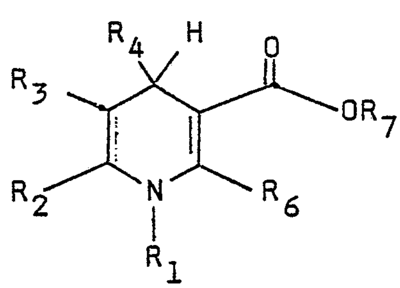

(3)

or a salt thereof
wherein
$R_1$ is H or lower alkyl;
$R_2$ and $R_6$ are each independently lower alkyl, aryl, or arylalkyl;
$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl;
$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$;and
$R_7$ is an alcohol derivative of the general formula $HOR_8$ wherein $R_8$ is $-O(CH_2)_nR_9$ wherein n is 2 or 3 and $R_9$ is NR′R″ wherein R′ is lower alkyl and R″ is arylalkyl, phenyl substituted in the para position with a 2,3-dihydroxypropoxy group, or phenoxy substituted in the para position with a 2-(tetrahydropyran-2-yloxy)ethyl group,
which comprises the following additional steps

a) reacting a compound of formula (1A) or (2A) with the appropriate alcohol derivative to form a compound of formula (3); and

b) optionally converting a compound of formula (3) to a salt.

14. The process of Claim 13 wherein the compound of formula (3) is selected from: 2,6-dimethyl-3-methoxycarbonyl-4-(3-nitrophenyl)-5-[2-(N-methyl-N-benzylamino)ethoxycarbonyl]-1,4-dihydropyridine, 2,6-dimethyl-3-methoxycarbonyl-4-(3-nitrophenyl)-5-(2-[4-(2,3-dihydroxypropoxy)phenyl]ethoxycarbonyl)-1,4-dihydropyridine;and 2,6-dimethyl-3-methoxycarbonyl-4-(3-nitrophenyl)-5-(3-[4-(2-tetrahydropyran-2-yloxy-ethyl)phenoxy]propoxycarbonyl)-1,4-dihydropyridine.

15. The use of the compounds prepared by the processes of claims 2 to 12 in the preparation of a 1,4-dihydropyridine derivative of the formula (3)

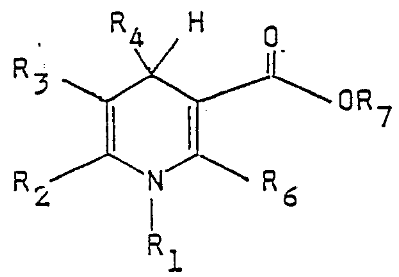

(3)

or a salt thereof
wherein
$R_1$ is H or lower alkyl;
$R_2$ and $R_6$ are each independently lower alkyl, aryl, or arylalkyl;
$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl;
$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$;and
$R_7$ is an alcohol derivative of the general formula $HOR_8$ wherein $R_8$ is $-O(CH_2)_nR_9$ wherein n is 2 or 3 and $R_9$ is NR′R″ wherein R′ is lower alkyl and R″ is arylalkyl, phenyl substituted in the para position with a 2,3-dihydroxypropoxy group, or phenoxy substituted in the para position with a 2-(tetrahydropyran-2-yloxy)ethyl group.

16. 1,4-Dihydropyridine derivatives of the formula (3)

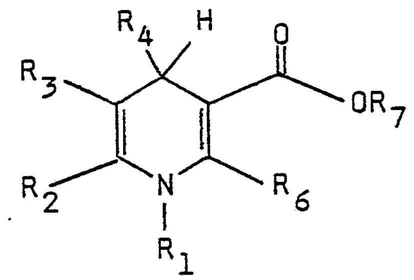

(3)

or a salt thereof
wherein
$R_1$ is H or lower alkyl;
$R_2$ and $R_6$ are each independently lower alkyl, aryl, or arylalkyl;
$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl;
$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$;and
$R_7$ is an alcohol derivative of the general formula $HOR_8$ wherein $R_8$ is $-O(CH_2)_nR_9$ wherein n is 2 or 3 and $R_9$ is NR′R″ wherein R′ is lower alkyl and R″ is arylalkyl, phenyl substituted in the para position with a 2,3-dihydroxypropoxy group, or phenoxy substituted in the para position with a 2-(tetrahydropyran-2-yloxy)ethyl group
containing detectable amounts of a resolving agent.

17. A compound of Claim 16 wherein said resolving agent comprises an optically active amine base such as cinchonidine, cinchonine, quinine, quinidine, strychnine, brucine, morphine, D-α-phenylethylamine, L-arginine, dehydroabietylamine, cinchonicine, L-2-amino-1-propanol, D-amphetamine, glucosamine, conessine, anabasine, D-ephedrine, or an N-alkyl-D-glucamine wherein alkyl is C1 to C10.

18. 1,4-Dihydropyridine derivatives of the formula (3)

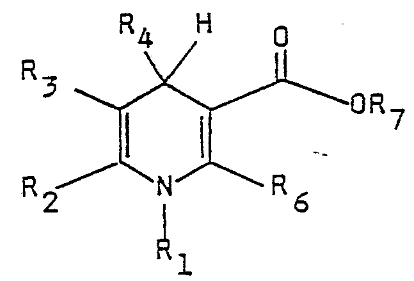

(3)

or a salt thereof
wherein
$R_1$ is H or lower alkyl;
$R_2$ and $R_6$ are each independently lower alkyl, aryl, or arylalkyl;
$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl;
$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$;and
$R_7$ is an alcohol derivative of the general formula $HOR_8$ wherein $R_8$ is $-O(CH_2)_nR_9$ wherein n is 2 or 3 and $R_9$ is NR′R″ wherein R′ is lower alkyl and R″ is arylalkyl, phenyl substituted in the para position with a 2,3-dihydroxypropoxy group, or phenoxy substituted in the para position with a 2-(tetrahydropyran-2-yloxy)ethyl group
containing detectable amounts of a compound of formula (1) or (2)

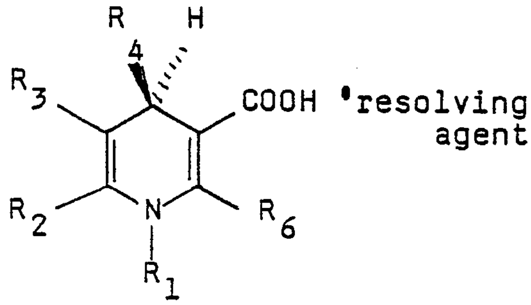

(1)

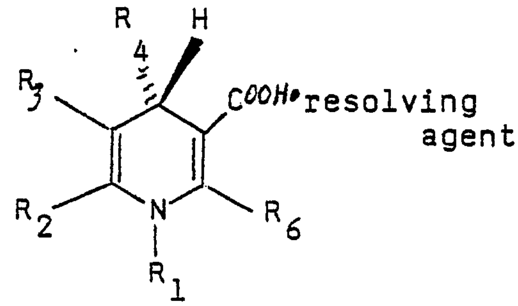

(2)

or a salt thereof
wherein

$R_1$ is H or lower alkyl;

$R_2$ and $R_6$ are each independently lower alkyl, aryl, or arylalkyl;

$R_3$ is CN, $No_2$, $CO_2R_5$, $CONHR_5$, $SO_2R_5$, or $P(O)(OR_5)_2$, where $R_5$ is lower alkyl, lower alkoxyalkyl, aryl, or arylalkyl;

$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$; and

resolving agent comprises an optically active amine ase such as cinchonidine, cinchonine, quinine, quinidine, strychnine, brucine, morphine, D-α-phenylethylamine, L-arginine, dehydroabietylamine, cinchonicine, L-2-amino-1-propanol, D-amphetamine, glucosamine, conessine, anabasine, D-ephedrine, or an N-alkyl-D-glucamine wherein alkyl is C1 to C10.

19. A compound of formula (1) or (2)

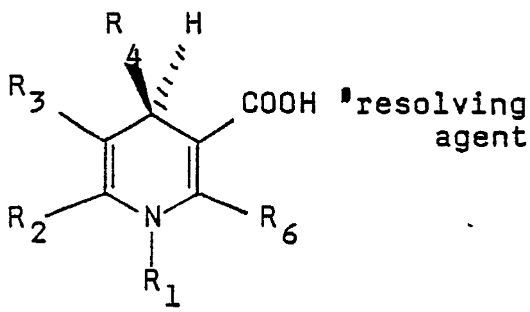

(1)

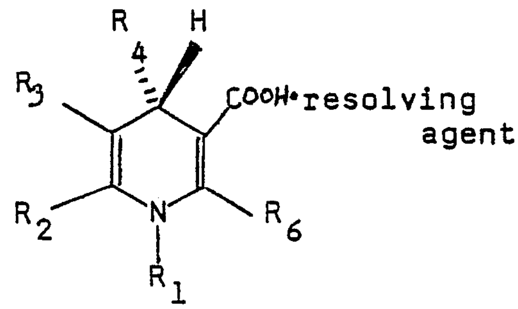

(2)

or a salt thereof
wherein

$R_1$ is H or lower alkyl;

$R_2$ and $R_6$ are each independently lower alkyl, aryl, or arylalkyl;

$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl;

$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$; and

resolving agent comprises an optically active amine base such as cinchonidine, cinchonine, quinine, quinidine, strychnine, brucine, morphine, D-α-phenylethylamine, L-arginine, dehydroabietylamine, cinchonicine, L-2-amino-1-propanol, D-amphetamine, glucosamine, conessine, anabasine, D-ephedrine, or an N-alkyl-D-glucamine wherein alkyl is C1 to C10 in admixture with a 1,4-dihydropyridine derivative of the formula (3)

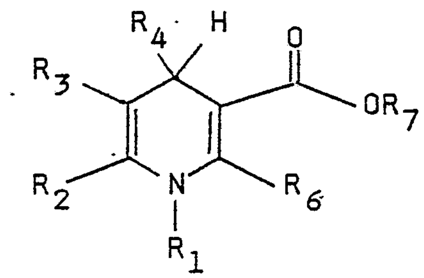

(3)

or a salt thereof
wherein

$R_1$ is H or lower alkyl;

$R_2$ and $R_6$ are each independently lower alkyl, aryl, or arylalkyl;

$R_3$ is CN, $NO_2$, $CO_2R_5$, $CONHR_5$, $SO_2R_5$, or $P(O)(OR_5)_2$, where $R_5$ is lower alkyl, lower alkoxyalkyl, aryl, or arylalkyl;

$R_4$ is aryl, heterocyclyl, or fused-ring heterocyclyl, optionally substituted with one, two, or three substituents independently selected from halo, $NO_2$, CN, lower alkyl, lower alkoxy, lower alkylamino, $CF_3$, $OCH_2F$, and $OCF_3$;and

$R_7$ is an alcohol derivative of the general formula $HOR_8$ wherein $R_8$ is $-O(CH_2)_nR_9$ wherein n is 2 or 3 and $R_9$ is NR'R" is lower alkyl and R" is arylalkyl, phenyl substituted in the para position with a 2,3-dihydroxypropoxy group, or phenoxy substituted in the para position with a 2-(tetrahydropyran-2-yloxy)ethyl group.